# EUROPÄISCHE PATENTSCHRIFT

(11) **EP 1 214 450 B1**
(45) Veröffentlichungstag und Bekanntmachung des Hinweises auf die Patenterteilung: **09.08.2006**
(21) Anmeldenummer: 00960634.4
(22) Anmeldetag: 08.09.2000
(51) Int. Cl.: C12Q 1/68

(54) **VERFAHREN UND NUKLEINSÄUREN ZUM NACHWEIS VON BRAUEREIRELEVANTEN MIKROORGANISMEN**
METHOD AND NUCLEIC ACIDS FOR DETERMINING THE PRESENCE OF MICRO-ORGANISMS SPECIFIC TO THE BREWING PROCESS
PROCEDE ET ACIDES NUCLEIQUES SERVANT A DETERMINER LA PRESENCE DE MICRO-ORGANISMES PRESENTANT UN INTERET DANS LE DOMAINE DE LA BRASSERIE

(30) Priorität: 24.09.1999 DE 19945964
(43) Veröffentlichungstag der Anmeldung: 19.06.2002
(73) Patentinhaber: Biotecon Diagnostics GmbH, 10589 Berlin (DE)
(72) Erfinder: FANDKE, Markus, 13353 Berlin (DE); GASCH, Alexander, 65239 Hochheim/Mainz (DE); BERGHOF, Kornelia, 12355 Berlin (DE)
(74) Vertreter: Grünecker, Kinkeldey, Stockmair & Schwanhäusser Anwaltssozietät
(86) Internationale Anmeldenummer: PCT/EP2000/008808
(87) Internationale Veröffentlichungsnummer: WO 2001/023605

(56) Entgegenhaltungen:
- WO-A-99/22023
- DE-A- 19 616 750
- US-A- 5 484 909
- SATOKARI REETTA ET AL: "Detection of beer spoilage bacteria Megasphaera and Pectinatus by polymerase chain reaction and colorimetric microplate hybridization." INTERNATIONAL JOURNAL OF FOOD MICROBIOLOGY, Bd. 45, Nr. 2, 8. Dezember 1998 (1998-12-08), Seiten 119-127, XP001002138 ISSN: 0168-1605
- PATENT ABSTRACTS OF JAPAN vol. 017, no. 278 (C-1065), 28. Mai 1993 (1993-05-28) & JP 05 015400 A (SAPPORO BREWERIES LTD), 26. Januar 1993 (1993-01-26)

### SEQUENZPROTOKOLL

<110> BioteCon Diagnostics GmbH
<120> Verfahren und Nukleinsäuren zum Nachweis von brauereirelevanten Mikroorganismen
<130> PCT1218-066
<140>
   <141>
<160> 107
<170> PatentIn Ver. 2.1
<210> 1
   <211> 267
   <212> DNA
   <213> Lactobacillus brevis
<400> 1
   tatatggaag taagacccct gagagatgat caggtagata ggctggaagt agcagcgccg 60
   tgaggcgtgg agcggaccag tactaatcgg tcgaggactt aaccaagtca acaacgtagt 120
   tgtttcgaga ataattgaat aatatctagt tttgagggaa gaagttctct tatagtgtgg 180
   tggcgatagc ctgaaggata cacctgttcc catgccgaac acagaagtta agcttcagca 240
   cgccgatagt agttggggga tcgcccc                                     267
<210> 2
   <211> 326
   <212> DNA
   <213> Lactobacillus lindneri
<400> 2
   ccattcctat atggaagtaa gactcctgaa agatgatcag gtcgataggt tagaagtgga 60
   agcatagtga tatgtgaagc ggactaatac taatcagtcg aggacttaac caaggaagac 120
   acagggttaa atcaaagttg aacagagaag atattatcta gttttgagag aacgaagttc 180
   gctcaggctt atgaaaaata agcatagtgt ggtggcgata gcctgaagga tacacctgtt 240
   cccatgccga acacagaagt taagcttcag cacgccaaaa gtagttgggg gatcgccccc 300
   tgcgaggata ggacgatggt catagc                                      326
<210> 3
   <211> 351
   <212> DNA
   <213> Lactobacillus casei
<400> 3
   ccattcctat atggaagtaa gacccctgag agatgatcag gtagataggc tggaagtgga 60
   agtgcagcga tgcatggagc ggaccagtac taatcggtcg aggacttaac caagtagagc 120
   gtgagcagga gcgcttagaa accggagcat aagcgggcct gagttcgttg gccgggtttt 180
   ggccaatgga ttcagggttc ttatgtggag gtttctgcga ctgcgaacgc gtttcgatga 240
   aatacactgg ttcccgacaa cacaaaaaca acaatgatag ccagttttga gagcgcaaag 300
   ttctcataag tgtggtggcg atagcaagaa ggatacacct gttcccatgc c          351
<210> 4
   <211> 414
   <212> DNA
   <213> Lactobacillus paracasei
<400> 4
   ccattcctat atggaagtaa gacccctgag agatgatcag gtagataggc tggaagtgga 60
   agtgcagcga tgcatggagc ggaccagtac taatcggtcg aggacttaac caagtaagag 120
   tgtgagcagg agcggttaga aaccggagca taagcgggcc tgagcgtgat ggccgggctt 180
   tggccattgc ggtcagggtc cttatgtgca ggtttctgcg actgcgaaca cgtttcgatg 240
   acaagtacgt taagttcaag gcagcaatta aacaatgata gctagttttg agagcgcaaa 300
   gttctcataa gtgtggtggc gatagcaaga aggatacacc tgttcccatg ccgaacacag 360
   aagttaagct tcttcacgcc gagagtagtt ggtgggaaac tgcctgcgag gata       414
<210> 5
   <211> 338
   <212> DNA
   <213> Lactobacillus paracasei
<400> 5
   ccattcctat atggaagtaa gacccctgag agatgatcag gtagataggc tggaagtgga 60
   agtgcagcga tgcatggagc ggaccagtac taatcggtcg aggacttaac caagtaagcg 120
   tgcaagcagg agcaggtttc tgcgactgcg aacacatttc gatgacaagt acgttaagtt 180
   caaggcagca attaaacgat gatagccagt tttgagagcg caaagttctc ataagtgtgg 240
   tggcgatagc aagaaggata cacctgttcc catgccgaac acagaagtta agcttcttca 300
   cgccgagagt agttggtggg aaactgcctg cgaggata                          338
<210> 6
   <211> 317
   <212> DNA
   <213> Lactobacillus coryniformis ssp. coryniformis
<400> 6
   ctcgagttga gatttcccat tcctttatgg aagtaagacc cctgagagat gatcaggtag 60
   ataggttgga agtggacgtg ccgtgaggca tggagcggac caatactaat cggtcgagga 120
   cttaaccaag tagcatgtac gtagtgttag tttaagggca aagaaatgaa tatccagttt 180
   tgagagcgca acgttctcag aaagtggtgt ggtggcgata gcaagaagga tacacctgtt 240
   cccatgtcga acacagaagt taagcttctt agcgccgaga gtagttgggg gagcaccccc 300
   tgcgaggata ggacgat                                                 317
<210> 7
   <211> 317
   <212> DNA
   <213> Lactobacillus coryniformis ssp. torquens
<400> 7
   ctcgagatga gatttcccat tcctttatgg aagtaagacc cctgagagat gatcaggtag 60
   ataggttgga agtggacgtg ccgtgaggca tggagcggac caatactaat cggtcgagga 120
   cttaaccaag tagcatgtac gtggtgttag tttaagggca aagaaatgaa tatccagttt 180
   tgagagcgca acgttctcag aaagtggtgt ggtggcgata gcaagaagga tacacctgtt 240
   cccatgtcga acacagaagt taagcttctt agcgccgaga gtagttgggg gagcaccccc 300
   tgcgaggata ggacgat                                                 317
<210> 8
   <211> 336
   <212> DNA
   <213> Lactobacillus curvatus
<400> 8
   acgcctcgag atgagatttc ccattccttt atggaagtaa gacccctgaa agatgatcag 60
   gtagataggc taggagtgga agtacagcga tgtatggagc ggactagtac taatcggtcg 120
   aggacttaac caaaggtgca atgttaggct tttgaaatga aatattactt attatgcagt 180
   tttgagagaa cgaagttctt ctcagtgcgc aagcacaaaa tagtgtggtg gcgatagcaa 240
   gaaggataca cctgttccca tgtcgaacac agaagttaag cttcttagcg ccgatagtag 300
   ttggtgggaa actacctgcg aggataggac gatggt                           336
<210> 9
   <211> 335
   <212> DNA
   <213> Pediococcus damnosus
<400> 9
   gatgagattt cccattccat ttatggaagt aagacccctg agagatgatc aggtagatag 60
   gttgggagtg gaagtgtagt gatacatgga gcggaccaat actaatcggt cgaggactta 120
   accacaaagt ggtgttctca agagaaggat tcgatattat ttagttttga gagaataaat 180
   ttctttcaca cgagccgcgt aagtggatcg gagaagtgtg gtgacgatag tgagaaggat 240
   acacctgttc ccatgtcgaa cacagaagtt aagcttctta acgccgagag tagttggggg 300
   atcgctccct gcgaggatag gacgatggtc aatag                            335
<210> 10
   <211> 326
   <212> DNA
   <213> Pediococcus inopinatus
<400> 10
   agatgagatt tcccattcca tttatggaag taagacccct gagagatgat caggtagata 60
   ggttgggagt ggaagtgtag tgatacatgg agcggaccaa tactaatcgg tcgaggactt 120
   aaccacaaag tggtgttctc aaagagaaga tttcgatatt atttagtttt gagagaataa 180
   atttctttca cacgagccgc ggaagtggat cggagaagtg tggtgacgat agtgagaagg 240
   atacacctgt tcccatgtcg aacacagaag ttaagcttct taacgccgag agtagttggg 300
   ggatcgctcc ctgcgaggat aggacg                                      326
<210> 11
   <211> 403
   <212> DNA
   <213> Pectinatus cerevisiiphilus
<400> 11
   aagtgctgaa agcatctaag cgtgaaacct gccttaagat gaggtttccc agagccgtaa 60
   ggcttggaag gcaccttgaa taagacgagg tagataggcc gggagtagaa gtacagtaat 120
   gtacgaagcg gactggtact aataagccga gagcttaact taaaatcatc gaaaaaaatg 180
   tttggtctga gatttcttct gtgaagtttt gagtgtgcaa gacactctgg ttgaagggca 240
   gggaacgtga gagcgtaaaa ctgcggactt tggctcaaag agttaaagca tctggtgacg 300
   atacctggat ggatccacct gttcccattc cgaacacagt agttaagcat ccacaggctg 360
   aaggtacttg gggggcgacc ccctgggaaa ataggacact gcc                   403
<210> 12
   <211> 434
   <212> DNA
   <213> Pectinatus frisingensis
<400> 12
   aagtgctgaa agcatctaag cgtgaaacca gctttaagat gaggtttccc agaacgcaag 60
   tttggaaggc accttgaaga agacgaggta gataggccgg gagtggaagt atggtgacat 120
   atgaagcgga ctggtactaa taagccgaga gcttaacttg atttcatcaa aaaagagaaa 180
   tgtttggtca gagattttct tctgtgaagt tttgagtgtg caagaacact cgagagtata 240
   taggtaaagg aaaagcagca gataagtttc ctggttactg tatataccgg ctgaggtgct 300
   gaggcactga aggccagaac atctggtggc gatacctgga tggatccacc tgttcccatt 360
   ccgaacacag tagttaagca tccacaggcc gaaggtactt ggggggcagc cccctgcgaa 420
   aataggacac cgcc                                                   434
<210> 13
   <211> 641
   <212> DNA
   <213> Pectinatus spec. DSM20764
<400> 13
   aagtgctgaa agcatctaag cgtgaaacct gccttaagat gaggtttccc agagccgtaa 60
   ggcttggaag gcaccttgaa gatgacgagg tagataggcc gggagtagaa gtatggtgac 120
   atacgaagcg gactggtact aataagccga gagcttaact taatttcatc tataaatgtt 180
   tggtcctgat ttcttctgtg aagttttgag tgtgcaagat cactcatgaa agtatatagg 240
   taaagggaaa gcagcagatt agttcctggt ttactttata tatgagcact aaggtgcaga 300
   aaagaacgtt tgaggaaacg cggcgttcgt aaactcactt tgcgtgctga ttatctcaat 360
   gctaaagcat taagataatt ttagaggaaa cgcgcgttca ctagcgttca ctctgcgtac 420
   tttatttcta agtgctgaag cactaagaag ggcaaggaaa cgcgtcgttc gcgatgctca 480
   ctttgcgtac ttcatctcta gactgctaaa gcagtaagat ctgaagcatc tggtggcgat 540
   acctggatgg atccacctgt tcccattccg aacacagtag ttaagcatcc acaggccgaa 600
   ggtacttggg gggcagcccc ctgcgagagt aggacatcgc c                     641
<210> 14
   <211> 495
   <212> DNA
   <213> Pectinatus spec. DSM20764
<400> 14
   aagtgctgaa agcatctaag cgtgaaacct gccttaagat gaggtttccc agagccgtaa 60
   ggcttggaag gcaccttgaa gatgacgagg tagataggcc gggagtagaa gtatggtgac 120
   atacgaagcg gactggtact aataagccga gagcttaact taatttcatc tataaatgtt 180
   tggtcctgat ttcttctgtg aagttttgag tgtgcaagat cactcatgaa agtatatagg 240
   taaaggggaa gcagattagt tcctggttta ctttatatat gagcactaag gtgcagaaaa 300
   gaacgtctaa ggaaacgcgg cgttcgtagg ctcactctgc gtacttcatc tctagactgc 360
   taaagcagta agatctgaag catctggtgg cgatacctgg atggatccac ctgttcccat 420
   tccgaacaca gtagttaagc atccacaggc cgaaggtact tggggggcag ccccctgcga 480
   aagtaggaca ccgcc                                                    495
<210> 15
   <211> 546
   <212> DNA
   <213> Megasphaera cerevisiae
<400> 15
   gcatctaagc gtgaaaccag cctagagatg aggtttctca ttacgaaagt aagtaaggtc 60
   ccatgaagac gacatggtag ataggccggg agtggacgta cagtaatgta tggagcggac 120
   cggtactaat agaccgagga cttgacttaa gcagggaacc cattttaaag aagcgaagcg 180
   acgcataaaa tggagtgagt cgcttatacc gaatcgcaga ttcggtaaag cagcggagaa 240
   taccaatgca gcggcaacac cagttagcat aaactaagcg gattcggagt gggtgaggga 300
   gtttcgtagc agcgtaggct aacccaacca ccgctttcga agaaggcgaa tggtttgaaa 360
   aagagtacat gcgaagaaac gacgaaagac tcacaaccaa aacatacaaa ctaagtagat 420
   gacattagag tcacaccgat tgttaagatc cgaaatactt ttcgatgtag ttgtcaggat 480
   acgaatcctg aaacgaattc agtggtgatg gctgcaggga tccacctgtt cccataccga 540
   acacag                                                             546
<210> 16
   <211> 306
   <212> DNA
   <213> Megasphaera cerevisiae
<400> 16
   gcatctaacc gtgaaaccag cctagagatg aggtttctca ttacgaaagt aagtaaggtc 60
   ccatgaagac gacatggtag ataggccggg agtggacgta cagtaatgta tggagcggac 120
   cggtactaat agaccgagga cttgacttaa gcaaagaagc aatagaaaga accatgtaga 180
   tggtgtaaga gttagacggg tagttaaggt ccgaaatact tttcgatgta gttgtcagga 240
   tacgaatcct gaaacgaatt cagtggtgat ggctgcaggg accacctgtt cccataccga 300
   acacag                                                             306
<210> 17
   <211> 449
   <212> DNA
   <213> Selenomonas laccicifex
<400> 17
   aagtgctgaa agcatctagg cgtgaagcct gtcccgagat gaagtatctc atggagtaat 60
   ccagtaagat tccttgaaga agacaaggta gataggttgg gagtgtaagc atcgtaaggt 120
   gttcagcgga ccaatactaa taaatcgagg gcttaacttt acagacctgt ccaagaagcg 180
   aagcggattg ggtaacaggt cgtatgcgaa aacatcccaa gaatcgagtc cgaagggcga 240
   agatgattgg cagatgttga ccgctaataa tctagaatgt ttcgatacaa tttttcttct 300
   gtatagtttt gagtggacat cgttcattca ataatatcca gtgacgatag ctgagtggta 360
   ccacctgttc ccataccgaa cacagtagtt aagcactcat acgccgaaag tacttgtctg 420
   gaaacgggct gcgagaatag gacgtcgcc 449
<210> 18
   <211> 343
   <212> DNA
   <213> Selenomonas lacticifex
<400> 18
   aagtgctgaa agcatctaag cgtgaagcct gtcccgagat gaagtatctc atggagtaat 60
   ccagtaagat tccttgaaga agacaaggta gataggttgg gagtgtaagc atcgtaaggt 120
   gttcagcgga ccaatactaa taaatcgagg gcttatctta ataatctaga atgtttcgat 180
   acaatttttc ttctgtatag ttttgagtgg acatggttca ttcaataata tccagtgacg 240
   atagctgagt ggtaccacct gttcccatac cgaacacagt agttaagcac tcatacgccg 300
   aaagtacttg tctggaaacg ggctgcgaaa ataggacgcc gcc 343
<210> 19
   <211> 395
   <212> DNA
   <213> Zymophilus raffinosivorans
<400> 19
   aagtgctgaa agcatctaag cgtgaaacca gccttaagat gaggtttctc acagagcaat 60
   ctggtaagac cccttgaaga agacaaggta gataggtcgg gagtggaagc gcagtaatgt 120
   gtgcagcgga ccgatactaa taggtcgagg gcttgactta aagccagaac gaaaactaaa 180
   atgcgaacat ttctttcttc tgtatagttt tgagagaaca aactcttaag atggagtagt 240
   ctgaggcgaa agcggaaggc agcgatatct aaaaaaagaa tatctggtag tgatagccaa 300
   gtggacccac ctgttcccat accgaacaca gtagttaagc acttgaacgt cgaaagtact 360
   tgggtggaaa cgccctgcga aaataggaca ccgcc 395
<210> 20
   <211> 395
   <212> DNA
   <213> Zymophilus paucivorans
<400> 20
   aagtgctgaa agcatctaag cgtgaaacca gccttaagat gaggtttctc acagagcaat 60
   ctggtaagac cccttgaaga agacaaggta gataggtcgg gagtggaagc gcagtaatgt 120
   gtgtagcgga ccgatactaa taggtcgagg gcttgactta aagccagaac gaattctaaa 180
   atgcgaacat ttctttcttc tgtatagttt tgagagaaca gactcttaag atgagcagtc 240
   tgaggcgaaa gctaaaggca gcgatatcta aaaaaaagaa tatctggtag tgatagccaa 300
   gtggacccac ctgttcccat accgaacaca gtagttaagc acttgaacgt cgaaagtact 360
   tgggtggaaa cgccctggga aaataggaca ccgcc 395
<210> 21
   <211> 21
   <212> DNA
   <213> Künstliche Sequenz
<220>
   <223> Beschreibung der künstlichen Sequenz: spezifische Sequenz für Lactobacillus brevis
<400> 21
   ccaagtcaac aacgtagttg t 21
<210> 22
   <211> 23
   <212> DNA
   <213> Künstliche Sequenz
<220>
   <223> Beschreibung der künstlichen Sequenz: spezifische Sequenz für Lactobacillus lindneri
<400> 22
   gacacagggt taaatcaaag ccg 23
<210> 23
   <211> 20
   <212> DNA
   <213> Künstliche Sequenz
<220>
   <223> Beschreibung der künstlichen Sequenz: spezifische Sequenz für Lactobacillus casei und Lactobacillus paracasei
<400> 23
   aggttzctcic gactgcgaac 20
<210> 24
   <211> 25
   <212> DNA
   <213> Künstliche Sequenz
<220>
   <223> Beschreibung der künstlichen Sequenz: spezifische Sequenz für Lactobacillus coryniformis
<400> 24
   atgtacgtag tgttagttta agggc 25
<210> 25
   <211> 20
   <212> DNA
   <213> Künstliche Sequenz
<220>
   <223> Beschreibung der künstlichen Sequenz: spezifische Sequenz für Lactobacillus curvatus
<400> 25
   cttczcagtg cgcaagcaca 20
<210> 26
   <211> 22
   <212> DNA
   <213> Künstliche Sequenz
<220>
   <223> Beschreibung der künstlichen Sequenz: spezifische Sequenz für Pediococcus damnosus
<400> 26
   gtgttctcaa gagaaggatt cg 22
<210> 27
   <211> 27
   <212> DNA
   <213> Künstliche Sequenz
<220>
   <223> Beschreibung der künstlichen Sequenz: spezifische Sequenz für Pediococcus inopinatus
<400> 27
   gttctcaaag agaagatttc gatatta 27
<210> 28
   <211> 23
   <212> DNA
   <213> Künstliche Sequenz
<220>
   <223> Beschreibung der künstlichen Sequenz: spezifische Sequenz für Pectinatus cerevisiiphilus
<400> 28
   tgagagcgta aaactgcgga ctt 23
<210> 29
   <211> 22
   <212> DNA
   <213> Künstliche Sequenz
<220>
   <223> Beschreibung der künstlichen Sequenz: spezifische Sequenz für Pectinazus frisingensis
<400> 29
   cagataagtt tcctggttac tg 22
<210> 30
   <211> 23
   <212> DNA
   <213> Künstliche Sequenz
<220>
   <223> Beschreibung der künstlichen Sequenz: spezifische Sequenz für Pectinatus spec. DSM 20764
<400> 30
   cactaaggtg cagaaaagaa cgt 23
<210> 31
   <211> 26
   <212> DNA
   <213> Künstliche Sequenz
<220>
   <223> Beschreibung der künstlichen Sequenz: spezifische Sequenz für Megasphaera cerevisiae
<400> 31
   cttttcgatg tagttgtcag gatacg 26
<210> 32
   <211> 25
   <212> DNA
   <213> Künstliche Sequenz
<220>
   <223> Beschreibung der künstlichen Sequenz: spezifische Searuenz für Selenomonas lacticifex
<400> 32
   gttcattcaa taatatccag tgacg 25
<210> 33
   <211> 23
   <212> DNA
   <213> Künstliche Sequenz
<220>
   <223> Beschreibung der künstlichen Sequenz: spezifische Sequenz für Zymophilus raffinosivorans
<400> 33
   aactcttaag acggagyagt ctg 23
<210> 34
   <211> 22
   <212> DNA
   <213> Künstliche Sequenz
<220>
   <223> Beschreibung der künstlichen Sequenz: spezifische Sequenz für Zymophilus paucivorans
<400> 34
   actcttaaga tgagcagtct ga 22
<210> 35
   <211> 21
   <212> DNA
   <213> Künstliche Sequenz
<220>
   <223> Beschreibung der künstlichen Sequenz: spezifische Sequenz für die Gattung Pediococcus
<400> 35
   agtstagtga tacatggagc g 21
<210> 36
   <211> 22
   <212> DNA
   <213> Künstliche Sequenz
<220>
   <223> Beschreibung der künstlichen Sequenz: spezifische Sequenz für die Gattung Pectinatus
<400> 36
   gtgaagtttt gagtgtgcaa ga 22
<210> 37
   <211> 22
   <212> DNA
   <213> Künstliche Sequenz
<220>
   <223> Beschreibung der künstlichen Sequenz: spezifische Sequenz für die Gattung Megasphaera
<400> 37
   gaccgaggac ttgacttaag ca 22
<210> 38
   <211> 20
   <212> DNA
   <213> Künstliche Sequenz
<220>
   <223> Beschreibung der künstlichen Sequenz: spezifische Sequenz für die Gattung Selenomonas
<400> 38
   cccagtgacg atagctgagt 20
<210> 39
   <211> 25
   <212> DNA
   <213> Künstliche Sequenz
<220>
   <223> Beschreibung der künstlichen Sequenz: spezifische Sequenz für die Gattung Zymophilus
<400> 39
   aagaacatct ggtagtgara gccaa 25
<210> 40
   <211> 19
   <212> DNA
   <213> Künstliche Sequenz
<220>
   <223> Beschreibung der künstlichen Sequenz: Consensus-Sequenz
<400> 40
   gtcgtgagac agttcggtc 19
<210> 41
   <211> 21
   <212> DNA
   <213> Künstliche Sequenz
<220>
   <223> Beschreibung der künstlichen Sequenz: Consensus-Sequenz
<400> 41
   cytagtacga gaggaccggr r 21
<210> 42
   <211> 21
   <212> DNA
   <213> Künstliche Sequenz
<220>
   <223> Beschreibung der künstlichen Sequenz: Consensus-Sequenz
<400> 42
   gctaccctgg ggataacagg c 21
<210> 43
   <211> 21
   <212> DNA
   <213> Künstliche Sequenz
<220>
   <223> Beschreibung der künstlichen Sequenz: Consensus-Sequenz
<400> 43
   atcgacgggg aggtttssca c 21
<210> 44
   <211> 20
   <212> DNA
   <213> Künstliche Sequenz
<220>
   <223> Beschreibung der künstlichen Sequenz: Consensus-Sequenz
<400> 44
   cacctcgatg tcggctcrtc 20
<210> 45
   <211> 18
   <212> DNA
   <213> Künstliche Sequenz
<220>
   <223> Beschreibung der künstlichen Sequenz: Consensus-Sequenz
<400> 45
   ccaagggttg ggctgttc 18
<210> 46
   <211> 19
   <212> DNA
   <213> Künstliche Sequenz
<220>
   <223> Beschreibung der künstlichen Sequenz: Consensus-Sequenz
<400> 46
   aagggccatc rctcaacgg 19
<210> 47
   <211> 20
   <212> DNA
   <213> Künstliche Sequenz
<220>
   <223> Beschreibung der künstlichen Sequenz: Consensus- Sequenz
<400> 47
   aagtgctgaa agcatctaag 20
<210> 48
   <211> 23
   <212> DNA
   <213> Künstliche Sequenz
<220>
   <223> Beschreibung der künstlichen Sequenz: Consensus-Sequenz
<400> 48
   tgtgttcgii atgggaacag gtg 23
<210> 49
   <211> 23
   <212> DNA
   <213> Künstliche Sequenz
<220> <223> Beschreibung der künstlichen Sequenz: Consensus-Sequenz
<400> 49
   tgtgttcgga atgggaacag gtg 23
<210> 50
   <211> 23
   <212> DNA
   <213> Künstliche Sequenz
<220>
   <223> Beschreibung der künstlichen Sequenz: Consensus-Sequenz
<400> 50
   tgtgttcgaa atgggaacag gtg 23
<210> 51
   <211> 23
   <212> DNA
   <213> Künstliche Sequenz
<220>
   <223> Beschreibung der künstlichen Sequenz: Consensus-Sequenz
<400> 51
   tgtgttcggt atgggaacag gtg 23
<210> 52
   <211> 23
   <212> DNA
   <213> Künstliche Sequenz
<220>
   <223> Beschreibung der künstlichen Sequenz: Consensus-Sequenz
<400> 52
   tgtgttcgat atgggaacag gtg 23
<210> 53
   <211> 23
   <212> DNA
   <213> Künstliche Sequenz
<220>
   <223> Beschreibung der künstlichen Sequenz: Consensus-Sequenz
<400> 53
   tgtgttcggc atgggaacag gtg 23
<210> 54
   <211> 23
   <212> DNA
   <213> Künstliche Sequenz
<220>
   <223> Beschreibung der künstlichen Sequenz: Consensus-Sequenz
<400> 54
   tgtattcgac atgggaacag gtg 23
<210> 55
   <211> 19
   <212> DNA
   <213> Künstliche Sequenz
<220>
   <223> Beschreibung der künstlichen Sequenz: Consensus-Sequenz
<400> 55
   ggcrrygccc taytytcsc 19
<210> 56
   <211> 19
   <212> DNA
   <213> Künstliche Sequenz
<220>
   <223> Beschreibung der künstlichen Sequenz: Consensus- Sequenz
<400> 56
   ggcagtgtcc tactttccc 19
<210> 57
   <211> 19
   <212> DNA
   <213> Künstliche Sequenz
<220> <223> Beschreibung der künstlichen Sequenz: Consensus-Sequenz
<400> 57
   ggcagcgtcc tactttcgc 19
<210> 58
   <211> 19
   <212> DNA
   <213> Künstliche Sequenz
<220>
   <223> Beschreibung der künstlichen Sequenz: Consensus-Sequenz
<400> 58
   ggcagtgtcc tactttcgc 19
<210> 59
   <211> 19
   <212> DNA
   <213> Künstliche Sequenz
<220>
   <223> Beschreibung der künstlichen Sequenz: Consensus-Sequenz
<400> 59
   ggcagcgtcc tactttccc 19
<210> 60
   <211> 18
   <212> DNA
   <213> Künstliche Sequenz
<220>
   <223> Beschreibung der künstlichen Sequenz: Consensus-Sequenz
<400> 60
   gyttmrcttc yrdgttcg 18
<210> 61
   <211> 18
   <212> DNA
   <213> Künstliche Sequenz
<220>
   <223> Beschreibung der künstlichen Sequenz: Consensus-Sequenz
<400> 61
   gcttaacttc cgtgttcg 18
<210> 62
   <211> 18
   <212> DNA
   <213> Künstliche Sequenz
<220>
   <223> Beschreibung der künstlichen Sequenz: Consensus-Sequenz
<400> 62
   gcttaacttc tatcttcg 18
<210> 63
   <211> 18
   <212> DNA
   <213> Künstliche Sequenz
<220>
   <223> Beschreibung der künstlichen Sequenz: Consensus-Sequenz
<400> 63
   gcttaacttc tgtgttcg 18
<210> 64
   <211> 18
   <212> DNA
   <213> Künstliche Sequenz
<220>
   <223> Beschreibung der künstlichen Sequenz: Consensus-Sequenz
<400> 64
   gcttaacttc catgttcg 18
<210> 65
   <211> 18
   <212> DNA
   <213> Künstliche Sequenz
<220>
   <223> Beschreibung der künstlichen Sequenz: Consensus-Sequenz
<400> 65
   gcttaacttc cgggttcg 18
<210> 66
   <211> 18
   <212> DNA
   <213> Künstliche Sequenz
<220>
   <223> Beschreibung der künstlichen Sequenz: Consensus-Sequenz
<400> 66
   gcttaacttc taggttcg 18
<210> 67
   <211> 18
   <212> DNA
   <213> Künstliche Sequenz
<220>
   <223> Beschreibung der künstlichen Sequenz: Consensus-Sequenz
<400> 67
   gcttaacttc taggttcg 18
<210> 68
   <211> 18
   <212> DNA
   <213> Künstliche Sequenz
<220>
   <223> Beschreibung der künstlichen Sequenz: Consensus-Sequenz
<400> 68
   gcttaacttc caggttcg 18
<210> 69
   <211> 18
   <212> DNA
   <213> Künstliche Sequenz
<220>
   <223> Beschreibung der künstlichen Sequenz: Consensus-Sequenz
<400> 69
   gcttaacttc cgagttcg 18
<210> 70
   <211> 18
   <212> DNA
   <213> Künstliche Sequenz
<220>
   <223> Beschreibung der künstlichen Sequenz: Consensus-Sequenz
<400> 70
   gcttaacttc taagttcg 18
<210> 71
   <211> 18
   <212> DNA
   <213> Künstliche Sequenz
<220>
   <223> Beschreibung der künstlichen Sequenz: Consensus-Sequenz
<400> 71
   gcttaacttc tgagttcg 18
<210> 72
   <211> 18
   <212> DNA
   <213> Künstliche Sequenz
<220>
   <223> Beschreibung der künstlichen Sequenz: Consensus-Sequenz
<400> 72
   gcttaacttc caagttcg 18
<210> 73
   <211> 25
   <212> DNA
   <213> Künstliche Sequenz
<220>
   <223> Beschreibung der künstlichen Sequenz: spezifische Sequenz für Lactobacillus brevis
<400> 73
   tcgagaataa ttgaataata tctag 25
<210> 74
   <211> 20
   <212> DNA
   <213> Künstliche Sequenz
<220>
   <223> Beschreibung der künstlichen Sequenz: spezifische Sequenz für Lactobacillus brevis
<400> 74
   gagggaagaa gttctcttat 20
<210> 75
   <211> 23
   <212> DNA
   <213> Künstliche Sequenz
<220>
   <223> Beschreibung der künstlichen Sequenz: spezifische Sequenz für Lactobacillus lindneri
<400> 75
   aacagagaag atattatcta gtt 23
<210> 76
   <211> 42
   <212> DNA
   <213> Künstliche Sequenz
<220>
   <223> Beschreibung der künstlichen Sequenz: spezifische Sequenz für Lactobacillus lindneri
<400> 76
   ttgagagaac gaagttcgct caggcttatg aaaaataagc at 42
<210> 77
   <211> 45
   <212> DNA
   <213> Künstliche Sequenz
<220>
   <223> Beschreibung der künstlichen Sequenz: spezifische Sequenz für Lactobacillus casei
<400> 77
   ttcgttggcc gggttttggc caatggattc agggttctta tgtgg 45
<210> 78
   <211> 58
   <212> DNA
   <213> Künstliche Sequenz
<220>
   <223> Beschreibung der künstlichen Sequenz: spezifische Sequenz für Lactobacillus casei
<400> 78
   gcgtttcgat gaaatacact ggttcccgac aacacaaaaa caacaatgat agccagtt 58
<210> 79
   <211> 29
   <212> DNA
   <213> Künstliche Sequenz
<220>
   <223> Beschreibung der künstlichen Sequenz: spezifische Sequenz für Lactobacillus casei und Lactobacillus paracasei
<400> 79
   ttagaaaccg gagcataagc gggcctgag 29
<210> 80
   <211> 46
   <212> DNA
   <213> Künstliche Sequenz
<220>
   <223> Beschreibung der künstlichen Sequenz: spezifische Sequenz für Lactobacillus paracasei
<400> 80
   gcgtgatggc cgggctttgg ccattgcggt cagggtcctt atgtgc 46
<210> 81
   <211> 46
   <212> DNA
   <213> Künstliche Sequenz
<220>
   <223> Beschreibung der künstlichen Sequenz: spezifische Sequenz für Lactobacillus paracasei
<400> 81
   caagtacgtt aagttcaagg cagcaattaa acaatgatag ctagtt 46
<210> 82
   <211> 44
   <212> DNA
   <213> Künstliche Sequenz
<220>
   <223> Beschreibung der künstlichen Sequenz: spezifische Sequenz für Lactobacillus coryniformis
<400> 82
   aaagaaatga atatccagtt ttgagagcgc aacgttctca gaaa 44
<210> 83
   <211> 48
   <212> DNA
   <213> Künstliche Sequenz
<220>
   <223> Beschreibung der künstlichen Sequenz: spezifische Sequenz für Lactobacillus curvatus
<400> 83
   aggtgcaatg ttaggctttt gaaatgaaat attacttatt atgcagtt 48
<210> 84
   <211> 22
   <212> DNA
   <213> Künstliche Sequenz
<220>
   <223> Beschreibung der künstlichen Sequenz: spezifische Sequenz für Pediococcus damnosus
<400> 84
   gccgcgtaag tggatcggag aa 22
<210> 85
   <211> 22
   <212> DNA
   <213> Künstliche Sequenz
<220>
   <223> Beschreibung der künstlichen Sequenz: spezifische Sequenz für Pediococcus inopinatus
<400> 85
   gccgcggaag tggatcggag aa 22
<210> 86
   <211> 25
   <212> DNA
   <213> Künstliche Sequenz
<220>
   <223> Beschreibung der künstlichen Sequenz: Sequenz zum Nachweis von Pediococcus damnosus, Pediococcus inopinatus und Pediococcus parvulus
<400> 86
   gagagaataa atttctttca cacga 25
<210> 87
   <211> 39
   <212> DNA
   <213> Kiuistliche Sequenz
<220>
   <223> Beschreibung der künstlichen Sequenz: spezifische Sequenz für Pectinatus cerevisiiphilus
<400> 87
   aaaatcatcg aaaaaaatgt ttggtctgag atttcttct 39
<210> 88
   <211> 25
   <212> DNA
   <213> Künstliche Sequenz
<220>
   <223> Beschreibung der künstlichen Sequenz: spezifische Sequenz für Pectinatus cerevisiiphilus
<400> 88
   cactctggtt gaagggcagg gaacg 25
<210> 89
   <211> 39
   <212> DNA
   <213> Künstliche Sequenz
<220>
   <223> Beschreibung der künstlichen Sequenz: spezifische Sequenz für Pectinatus frisingensis
<400> 89
   gatttcatca aaaaagagaa atgtttggtc agagatttt 39
<210> 90
   <211> 33
   <212> DNA
   <213> Künstliche Sequenz
<220>
   <223> Beschreibung der künstlichen Sequenz: spezifische Sequenz für Pectinatus frisingensis
<400> 90
   tatataccgg ctgaggtgct gaggcactga agg 33
<210> 91
   <211> 36
   <212> DNA
   <213> Künstliche Sequenz
<220>
   <223> Beschreibung der künstlichen Sequenz: spezifische Sequenz für Pectinatus spec. DSM 20764
<400> 91
   aatttcatct ataaatgttt ggtcctgatt tcttct 36
<210> 92
   <211> 54
   <212> DNA
   <213> Künstliche Sequenz
<220>
   <223> Beschreibung der künstlichen Sequenz: spezifische Sequenz für Pectinatus spec. DSM 20764
<400> 92
   agattagttc ctggtttact ttatatatga gcactaaggt gcagaaaaga acgt 54
<210> 93
   <211> 20
   <212> DNA
   <213> Künstliche Sequenz
<220>
   <223> Beschreibung der künstlichen Sequenz: spezifische Sequenz für Pectinatus spec. DSM 20764
<400> 93
   aggaaacgcg gcgttcgtaa 20
<210> 94
   <211> 56
   <212> DNA
   <213> Künstliche Sequenz
<220>
   <223> Beschreibung der künstlichen Sequenz: spezifische Sequenz für Selenomonas lacticifex
<400> 94
   taataatcta gaatgtttcg atacaatttt tcttctgtat agttttgagt ggacat 56
<210> 95
   <211> 24
   <212> DNA
   <213> Künstliche Sequenz
<220>
   <223> Beschreibung der künstlichen Sequenz: spezifische Sequenz für Zymophilus raffinosivorans
<400> 95
   gaggcgaaag cggaaggcag cgat 24
<210> 96
   <211> 24
   <212> DNA
   <213> Künstliche Sequenz
<220>
   <223> Beschreibung der künstlichen Sequenz: spezifische Sequenz für Zymophilus paucivorans
<400> 96
   gaggcgaaag ctaaaggcag cgat 24
<210> 97
   <211> 37
   <212> DNA
   <213> Künstliche Sequenz
<220>
   <223> Beschreibung der künstlichen Sequenz: spezifische Sequenz für Megasphaera cerevisiae
<400> 97
   aatcctgaaa cgaattcagt ggtgatggct gcaggga 37
<210> 98
   <211> 20
   <212> DNA
   <213> Künstliche Sequenz
<220>
   <223> Beschreibung der künstlichen Sequenz: Sequenz zum Nachweis brauereirelevanter Bakterien der Familie der Lactobacillaceae
<400> 98
   tatggaagta agacccctga 20
<210> 99
   <211> 21
   <212> DNA
   <213> Künstliche Sequenz
<220>
   <223> Beschreibung der künstlichen Sequenz: Sequenz zum Nachweis brauereirelevanter Bakterien der Familie der Lactobacillaceae
<400> 99
   agatgatcag gtagataggc t 21
<210> 100
   <211> 21
   <212> DNA
   <213> Künstliche Sequenz
<220>
   <223> Beschreibung der künstlichen Sequenz: Sequenz zum Nachweis brauereirelevanter Bakterien der Familie der Lactobacillaceae
<400> 100
   agatgatcag gtcgataggt t 21
<210> 101
   <211> 21
   <212> DNA
   <213> Künstliche Sequenz
<220>
   <223> Beschreibung der künstlichen Sequenz: Sequenz zum Nachweis brauereirelevanter Bakterien der Familie der Lactobacillaceae
<400> 101
   agatgatcag gtagataggt t 21
<210> 102
   <211> 25
   <212> DNA
   <213> Künstliche Sequenz
<220>
   <223> Beschreibung der künstlichen Sequenz: Sequenz zum Nachweis brauereirelevanter Bakterien der Familie der Lactobacillaceae
<400> 102
   tactaatcgg tcgaggactt aacca 25
<210> 103
   <211> 26
   <212> DNA
   <213> Künstliche Sequenz
<220>
   <223> Beschreibung der künstlichen Sequenz: Sequenz zum Nachweis brauereirelevanter Bakterien der Familie der Lactobacillaceae
<400> 103
   atactaatca gtcgaggact taacca 26
<210> 104
   <211> 32
   <212> DNA
   <213> Künstliche Sequenz
<220>
   <223> Beschreibung der künstlichen Sequenz: spezifische Sequenz für die Gattung Pectinacus
<400> 104
   gaagcggact ggtactaata agccgagagc tt 32
<210> 105
   <211> 32
   <212> DNA
   <213> Künstliche Sequenz
<220>
   <223> Beschreibung der künstlichen Sequenz: spezifische Sequenz für die Gattung Selenomonas
<400> 105
   cagcggacca atactaataa atcgagggct ta 32
<210> 106
   <211> 38
   <212> DNA
   <213> Künstliche Sequenz
<220>
   <223> Beschreibung der künstlichen Sequenz: spezifische Sequenz für die Gattung Zymophilus
<400> 106
   agcggaccga tactaatagg tcgagggctt gacttaaa 38
<210> 107
   <211> 32
   <212> DNA
   <213> Künstliche Sequenz
<220>
   <223> Beschreibung der künstlichen Sequenz: spezifische Sequenz für die Gattung Megasphaera
<400> 107
   ggagcggacc ggtactaata gaccgaggac tt 32

## Beschreibung

Die Erfindung betrifft ein Verfahren zum Nachweis von L. brevis sowie Nukleinsäuren und Kombinationen derselben die in diesem Verfahren eingesetzt werden können. Die Erfindung betrifft ferner die Verwendung der erfindungsgemäßen Nukleinsäuren oder Kombinationen derselben zum Nachweis und/oder zum Identifizieren und/oder Charakterisieren verschiedener Gattungen oder Arten von L. brevis.

Bier ist als mikrobiologisch sehr stabil anzusehen und kann nur von einer relativ überschaubaren Anzahl von Bakterien verdorben werden. Um eine Kontamination mit diesen Keimen möglichst frühzeitig zu entdecken, muß ein Analysesystem angewendet werden, das eine schnelle Detektion der Keime in der Matrix Bier ermöglicht, da sofort Gegenmaßnahmen ergriffen werden müssen.

Das gemeinsame Merkmal aller bierschädlichen Keime ist die Spurenkontamination einzelner Gebinde (Fässer. Flaschen) und das nur langsame Wachstum. Besonders die mikrobiologische Kultur der anaeroben Keime ist sehr problematisch. Die derzeit bekannten bierverderbenden Bakterien sind folgenden Gattungen zugeordnet *Lactobacillus, Pediococcus, Pectinatus, Megasphaera.* Vertreter der Gattungen *Selenomonas* und *Zymophilus* sind als Bierkontaminanten zwar noch nicht in Erscheinung getreten, eine Kontamination des Bieres und ihr anschließendes Wachstum darin ist jedoch nicht auszuschließen.

Die Gattung *Lactobacillus* beschreibt Gram-positive, nicht sporenbildende meist unbewegliche und kettenbildende Stäbchen, die lang, schlank und manchmal gebogen sind. Teilweise werden auch kokkoide Formen beobachtet. Vertreter der Gattung *Lactobacillus* sind mikroaerophil, z.T. anaerob. Sie sind cytochrom- und katalase negativ, der Stoffwechsel ist fermentativ und sie benötigen ein komplexes Nährmedium. Der molare G+C-Gehalt der DNA liegt zwischen 32 und 53%. *Lactobacillen* werden außer in Bier in Molkerei- und Getreideprodukten, in Fleisch und Fischprodukten, in Wasser, Abwasser, Wein, Früchten und Fruchtsäften, sauer eingelegten Gemüsen, Sauerkraut, Silage und Sauerteig gefunden. Sie sind zwar Bestandteil der normalen Mund-, Intestinal- und Vaginalflora von Säugern, jedoch selten pathogen (*Bergeys Manual of Syst. Microbiolology,* 1984, S. 1209-1234 ). In Bier führen sie durch ihre Stoffwechselprodukte zu Trübungen und unerwünschten Geschmacksveränderungen. Für den Bierverderb relevante Arten sind *Lactobacillus brevis, Lactobacillus lindneri, Lactobacillus casei, Lactobacillus paracasei, Lactobacillus coryniformis* und *Lactobacillus curvatus* (Back, *Brauwelt*, 1980, 120, S. 1562-1569).

Die Gattung *Pediococcus* umfaßt Gram-positive, unbewegliche und nichtsporenbildende Kokken. Sie bilden Tetraden oder liegen paarweise vor. Sie sind fakultativ anaerob, die Sauerstoffempfindlichkeit unterscheidet sich von Art zu Art. *Pediococcen* sind cytochrom- und katalase negativ und benötigen ein komplexes Nährmedium (*Bergeys Manual of Syst. Microbiolology,* 1984, S. 1075-1079). Sie werden als Starterkulturen zur Rohwurstherstellung eingesetzt, sie fermentieren verschiedene Sauergemüsearten und führen zum Verderb von Lebensmitteln (Fimhaber, Baumgart: *Mikrobiologische Untersuchung von Lebensmitteln,* 1993, S. 413-419,115-117). Die Gattung umfaßt 8 Spezies, die Arten *Pediococcus damnosus* und *Pediococcus inopinatus* sind als bierschädlich anzusehen.

Die Gattung *Pectinatus* umfaßt die Arten *Pectinatus cerevisiiphilus, Pectinatus frisingiensis* und den nicht weiter taxonomisch eingeordneten Stamm *Pectinatus sp.* DSM 20764. Alle Stämme wurden aus verdorbenem Bier isoliert (Schleifer et al, *Int. J. of Syst. Bacteriology,* 1990, S. 19-27). Es handelt sich um leicht gekrümmte, nicht sporenbildende stäbchenförmige Bakterien. Sie sind kammähnlich begeißelt und beweglich. Sie produzieren weder Katalase noch Cytochromoxidase und sind obligat anaerob. Der molare G+C-Gehalt beträgt 38-41 %. Die Zellwand der Gattung *Pectinatus,* aber auch die der Gattungen *Megasphaera, Selenomonas* und *Zymophilus,* ist den Gram-positiven Bakterien ähnlicher als den Gram-negativen Bakterien. Obwohl die Gram-Färbung negativ ist, werden sie taxonomisch den Gram-positiven Bakterien zugeordnet (Haikara, *The Prokaryotes,* 2. Ausgabe, Band 11, 1991, S. 1993-2004).

Die Gattung *Megasphaera* umfaßt die Arten *Megasphaera elsdenii* und *Megasphaera cerevisiae.* Nur *Megasphaera cerevisiae* ist brauereirelevant und wird als Gram-negative, strikt anaerobe, cytochrom- und katalasenegative, unbewegliche und teilweise leicht gestreckte Kokke beschrieben, die einzeln, in Paaren oder in kurzen Ketten vorliegt. Der mittlere Zelldurchmesser liegt bei 1,4 µm, der molare G+C-Gehalt bei 42,4-44,8%. Hauptstoffwechselprodukte sind Schwefelverbindungen wie H₂S und flüchtige Fettsäuren. Eine Kontamination mit *Megasphaera cerevisiae* führt in Bier zu sehr starken Aroma- und Geschmacksveränderungen (Haikara, *The Prokaryotes,* 2. Ausgabe, Band II, 1991, S. 1993-2004).

Spezies der Gattung *Selenomonas* sind definiert als obligat anaerobe, Gram-negative, nicht sporenbildende, leicht gebogene und bewegliche Stäbchen. Der molare G+C-Gehalt liegt bei 48-58% (Schleifer et al, *Int. J. of Syst. Bacteriology,* 1990, S. 19-27). *Selenomonaden* werden isoliert aus dem Magen und Darmtrakt und dem Kot von Säugem. Die Gattung umfaßt 10 Arten (Hespell et al., *The Prokaryotes,* 2. Ausgabe, Band II, 1991, S. 2005-2013). Nur *Selenomonas lacticifex* ist aus Anstellhefe isoliert worden und somit brauereirelevant. Zwar ist *Selenomonas lacticifex* noch nicht als bierverderbendes Bakterium in Erscheinung getreten, ein Wachstum in Bier ist jedoch möglich und somit die Definition eines bierverderbenden Organismus erfüllt.

Zu der Gattung *Zymophilus* gehören die Arten *Zymophilus paucivorans* und *raffinosivorans* als Gram-negätive, leicht gekrümmte, bewegliche Stäbchen, die einzeln, paarweise oder in kurzen Ketten auftreten. Der molare G+C-Gehalt liegt bei 38-41 %. Sie sind obligat anaerob und führen einen fermentativen Metabolismus. Beide Arten wurden aus Anstellhefen und Brauereiabfällen isoliert, ein Wachstum in Bier ist nur bei *Zymophilus raffinosivorans* beobachtet worden (Schleifer et al, *Int J. of Syst. Bacteriology*, 1990, S. 19-27).

Basierend auf dem Vergleich der 16S-rRNA-Gensequenzen werden alle zu untersuchenden Gattungen den Gram-positiven Bakterien mit niedrigem G+C-Gehalt zugeordnet. Die Gattungen *Pediococcus* und *Lactobacillus* werden in die Familie der *Lactobacillaceae* eingeordnet, die Gattungen *Pectinatus, Megasphaera, Selenomonas* und *Zymophilus* in die Sporomusa-Gruppe. Die Sporomusa-Gruppe wird auch als Gruppe der Gram-positiven Eubakterien mit Gram-negativer Zellwand bezeichnet (Stackebrandt et al., *The Prokaryotes,* 2. Ausgabe, Band II, 1991, S. 25-26, 33).

Eine klassische mikrobiologische Bestimmung der oben beschriebenen Keime kann bis zu 10 Tage in Anspruch nehmen. Wünschenswert ist jedoch eine deutlich schnellere Analyse, da ansonsten unnötige Lagerungskosten entstehen oder das untersuchte Bier schon ausgeliefert worden ist. Aus diesem Grunde sind bereits mehrere Schnellnachweisverfahren entwickelt worden. So können beispielsweise Bierschädlinge aufgrund ihrer Stoffwechselprodukte detektiert werden (Haikara et al., Micröbiology, 1995, 141, S. 1131-1137). Weitere indirekte Methoden sind die Turbidometrie (Haikara et al., *ASBC,* 1990, S. 92-95) und die Messung der ATP-Biolumineszenz (Miller et al., *J. Inst. Brew.,* 1989, Band 95, S. 317-319). Schnell und spezifisch ist auch der Nachweis mittels Antikörpem (Gares et al., *ASBC,* 1993, S. 158-163; Winnewisser et al., *Int. J. of Bacteriology,* 1995, 45, S. 403-405). Bei diesen Methoden liegt der Nachteil darin, daß entweder unspezifische Parameter untersucht werden oder nur jeweils eine Spezies oder Gattung nachgewiesen wird. Auch ist der apparative und personelle Aufwand groß. Einen Überblick über Schnellmethoden zum Nachweis von brauereirelevanten Kontaminationen gibt Dowhanick (*Cerevisia,* 1995, 20/4, S. 40-49).

Die Polymerase-Kettenreaktion ("polymerase chain reaction", PCR; Mullis et at., siehe US 4 683 195, US 4 683 202, US 4 965 188) ist eine schnelle und effektive Methode, Keime spezifisch nachzuweisen. Es sind eine Reihe von Nukleinsäuremolekülen bekannt, durch deren Verwendung als Primer und/oder Sonden der spezifische Nachweis von brauereirelevanten Mikroorganismen möglich ist. Nachteilig ist jedoch, daß bei Einsatz dieser Nukleinsäuremoleküle in einer Amplifikations- bzw. Detektionsreaktion immer nur ein Bruchteil aller möglichen brauereirelevanten Mikroorganismen nachgewiesen werden kann. Diese PCR Systeme dienen zum spezifischen Nachweis jeweils nur einzelner Spezies in einer Amplifikationsreaktion der Gattungen *Lactobacillus, Pediococcus, Pectinatus* und *Megasphaera* (Sakamoto US 5 869 642, Nietupski et al. US 5 705 339, US 5 484 909, Tsiuchia et al. JP06141899A, JP06113888A / *ASBC J.*, 1992, S. 64-67 / *ASBC J.* 1993, S. 40-41, Yasui JP07289295A / *Can. J. Microbiol.,* 1997 43, S. 157-163, Shimada et al. JP06090793, Alatossava et al. WO97/09448, Doyle et al., *J. of Ind. Microbiology,* 1995, 15, S. 67-70, DiMichele et al. *ASBC J.,* 1993, S. 63-66; Vogeser et al., *Brauwelt,* 1998, 24/25, S. 1060-1063). Ferner sind die beschriebenen Verfahren zur Visualisierung der entstandenen Amplifikate, wie etwa die Agarosegelelektrophorese, problematisch, da das cancerogene und hochgiftige Ethidiumbromid zur Anfärbung der Amplifikate verwendet wird. Diese Verfahren sind nur schwer automatisierbar und die Auswertung der Agarosegele bzw. die Identifizierung der Keime anhand der Arnplifikatgrößen ist z.T. nicht eindeutig.

JP 05 015 400 offenbart die Sequence eine Sonde zum gattungsspezifischen nachweis von Lactobazillen.

Der vorliegenden Erfindung lag die Aufgabe zugrunde, ein Verfahren sowie Mittel anzugeben, die einer Schneiltest auf Kontamination von Bier und Brauereirohstoffen durch Mikroorganismen des Spezies L. brevis ermöglichen.

Diese Aufgabe wird erfindungsgemäß gelöst durch ein Verfahren zum Nachweis von *Lactobacillus brevis* in einer Probe, das folgende Schritte umfasst:
(a) Inkontaktbringen der Probe mit einer Kombination aus mindestens zwei ersten Nukleinsäuremolekülen (Primem), die mit einem in allen Bakterien der Spezies Lactobacillus brevis, Lactobacillus lindneri, Lactobacillus casei, Lactobacillus paracasei, Lactobacillus coryniformis, Lactobacillus curvatus, Pediococcus damnosus, Pediococcus inopinafus, Pectinatus cerevisiiphilus, Pectinatus frisingiensis, Pectinatus sp. DSM 20764, Megasphaera cerevisiae, Selenomonas lacticifex, Zymophilus paucivorans, Zymophilus raffinosivorans konservierten Bereich einer mikrobiellen Nukleinsäure hybridisieren;
   wobei die mindestens 2 ersten Nukleinsäuremoleküle vom intergenischen Spacer 23S-5S bzw. den angrenzenden 23S- oder 5S-rDNA-Genregionen aus L. brevis abgeleitet, d.h. wie in SEQ ID NO:1, dargestellt,sind;
(b) Amplifizieren der mikrobiellen Nukleinsäure oder eines Teils derselben zur Erzeugung von mindestens einem Amplifikationsfragment;
(c) Inkontaktbringen der in Schritt (b) erhaltenen Amplifikationsfragmente mit mindestens einem zweiten Nukleinsäuremolekül (Sonde), welches mit mindestens einem Amplifikationsfragment spezifisch hybridisiert, das eine für Bakterien der Art *Lactobacillus brevis* spezfische Sequenz der mikrobiellen Nukleinsäure umfasst,
   wobei das mindestens eine zweite Nukleinsäuremolekül vom intergenischen Spacer 23S-5S bzw. den angrenzenden 23S- oder 5S-rDNA Genregionen aus L. brevis abgeleitet, d.h. wie in SEQ ID NO:1 dargestellt, ist;
(d) Nachweis von mindestens einer Hybridnukleinsäure, die aus einem Amplfikationsfragment und einem im Schritt (c) eingebrachten zweiten Nukleinsäuremolekül besteht.

Nukleinsäuremolekül geeignet als Sonde zum keimspezifischen Nachweis von Bakterien der Art *Lactobacillus brevis,* ausgewählt aus:
(i) einer Nukleinsäure mit einer Sequenz nach SEQ ID NO 21 oder SEQ ID NO 73-74 oder einem 15-30 Nukleotide langen Fragment von SEQ ID NO:1, 21 oder 73-74;
(ii) einer Nukleinsäure, die mindestens 90% identisch mit einer Nukleinsäure nach (i) ist, oder
(iii) einer Nukleinsäure, die komplementär zu einer Nukleinsäure nach (i) oder (ii) ist,
wobei das Nukleinsäuremolekül nur mit *L. brevis* spezifisch hybridisiert und nicht mit
Lactobacillus lindneri, lactobacillus casei, Lactobacillus paracasel, lactobacillus coryniformis coryniformis, Lactobacillus coryniformis torquens, Lactobacillus curvatus, Pediococcus damnosus, Pediococcus inopinatus, Pectinatus cerevisiiphilus, Pectinafus frisingiensis, Pectinatus sp. DSM 20462, Megasphaera cerevisiae, Selenomonas lacdcifex, Zymophilus paucivorans, Zymophilus raffinosivorans;
und die
Verwendung eines Nukleinsäuremoleküls ausgewählt aus
(a)
   (i) einer Nukleinsäure mit einer Sequenz nach SEQ ID NO 21 oder SEQ ID NO 73-74, oder einem Fragment von SEQ ID NO: 1, 21, 73 oder 74 mit 15-30 Nukleotiden,
   (ii) einer Nukleinsäure, die mindestens 90% identisch zu einer Nukleinsäure nach (i) ist, und
   (iii) einer Nukleinsäure, die komplementär zu einer Nukleinsäure nach (i) bis (ii) ist,
   wobei das Nukleinsäuremolekül nur mit *L. brevis* spezifisch hybridisiert und nicht mit Lactobacillus lindneri, Lactobacillus casei, Lactobacillus paracasei, Lactobacillus coryniformis coryniformis, Lactobacillus coryniformis torquens, Lactobacillus curvatus, Pediococcus damnosus, Pediococcus inopinatus, Pectinatus cerevisiiphilus, Pectinafus frisingiensis, Pectinatus sp. DSM 20462, Megasphaera cerevisiae, Selenomonas lacticifex, Zymophilus paucivorans, Zymophilus raffinosivorans
   zum keimspezifischen Nachweis und/oder Identifizieren und/oder Charakterisieren von Bakterien der Art *Lactobacillus brevis*; oder
(b) einer Kombination von mindestens 2 Nukleinsäuren von (a) zum keimspezifischen Nachweis von L. brevis.

In den Sequenzen nach SEQ ID NO 1-107 werden Nukleotide folgendermaßen abgekürzt: G = Guanosin, A = Adenosin, T = Thymidin, C = Cytidin, U = Uracil, i = Inosin. Nach IUPAC werden Mischungen wie folgt abgekürzt R = G oder A. Y = C oder T, K = G oder T, W = A oder T, S = C oder G, M = A oder C. B = C, G oder T, D = A, G oder T, H = A, C oder T, V = A, C oder G, N = A. C, G oder T.

Zur Bestimmung der Identität (im Sinne von vollständiger Übereinstimmung, entsprechend 100 % Identität) bei Nukleinsäure-Sequenzen nach (iii) werden Teilsequenzen eines größeren Polynukleotids betrachtet Diese Teilsequenzen umfassen 10 Nukleotide und sind dann identisch, wenn alle 10 Bausteine bei zwei Vergteichssequenzen identisch sind. Die Nukleotide Thymidin und Uridin sind als identisch anzusehen. Als Teilsequenzen können alle möglichen Fragmente eines größeren Polynukleotids betrachtet werden.

Dabei liegt 90 % Identität vor, wenn in den beiden zu vergleichenden Sequenzen in einem Abschnitt 9 von 10 Nukleotide bzw. 18 von 20 Nukleotide identisch sind.

Als Beispiel seien zwei Polynukleotide betrachtet, die 20 Nukleotide umfassen und sich in dem 5. Baustein unterscheiden. In einem Sequenzvergleich findet man dann sechs 10-er Nukleotide, die identisch sind und 5, die nicht identisch sind, da sie sich in einem Baustein unterscheiden.

Außerdem kann die Identität graduell bestimmt werden, wobei die Einheit in Prozent angegeben wird. Zur Bestimmung des Grades der Identität werden auch Teilsequenzen betrachtet die minimal die Länge der tatsächlich genutzten Sequenz, z.B. als Primer, oder aber 20 Nukleotide umfassen.

Als Beispiel werden Polynukleotide A mit einer Länge von 100 Nukleotiden und B mit eine Länge von 200 Nukleotiden verglichen. Aus Polynukleotid B wird ein Primer abgeleitet mit einer Länge von 14 Nukleotiden. Zur Bestimmung des Grades der Identität wird Polynukleotid A mit dem Primer in seiner ganzen Länge verglichen. Wenn die Sequenz des Primers in Polynukleotid A vorkommt, wobei sie aber in einem Baustein abweicht, dann gibt es ein Fragment mit einem Identitätsgrad von 13:14 → 92.3 %.

Im zweiten Beispiel werden die zuvor genannten Polynukleotide A und B in ihrer Gesamtheit verglichen. In diesem Fall werderralle möglichen Vergleichsfenster einer Länge von 20 Nukleotiden angelegt und für sie der Identitätsgrad bestimmt. Sind also Nukleotid Nr. 50-69 von Polynukleotid A und B mit Ausnahme von Nukleotid Nr. 55 identisch, dann ergibt sich für diese Fragmente ein identitätsgrad von 19:20 → 95 %.

Das erfindungsgemäße Verfahren ist schneller als bisherige mikrobiologische Nachweisverfahren durchführbar und erlaubt den keinspezifischen Nachweis von L. brevis. Mittels des erfindungsgemäßen Verfahrens kann L. brevis sowohl in Bierproben als auch in Rohstoffproben (Gerstenmalz, Hefe, Hopfen, Wasser) oder Proben von Zwischenprodukten in der Bierherstetlung (z.B. Maische, Würze) nachgewiesen werden, und zwar auch wenn die Anzahl der kontaminierenden Mikroorganismen noch gering ist.

Als brauereirelevante Mikroorganismen werden in diesem Zusammenhang in erster Linie Bakterien und insbesondere die oben beschriebenen Bakterien *Lactobacillus brevis, Lactobacillus lindneri, Lactobacillus casei, Lactobacillus paracasei, Lactobacillus coryniformis, Lactobacillus curvatus, Pediococcus damnosus, Pediococcus inopinatus, Pectinatus cerevisiiphilus, Pectinatus frisingiensis, Pectinatus sp.* DSM 20764, *Megasphaera cerevisiae, Selenomonas lacticifex, Zymophilus paucivorans* und *Zymophilus raffinosivorans* verstanden, sowie auch alle in Bier auffindbaren Mikroorganismen, die zwar nicht zu den vorgenannten Spezies gehören, dennoch sich in Bier vermehren können, beispielsweise seltene Vertreter der Familie der *Lactobacillaceae* wie *Lactobacillus malefermentans, Lactobacillus buchneri, Lactobacillus parabuchneri, Lactobacillus sanfrancisco, Lactobacillus delbrueckii, Leuconostoc mesenteroides, Pediococcus pentosacaeus* und *Lactococcus lactis.*

Die durch das erfindungsgemäße Verfahren nachweisbaren Mikroorganismen sind also nicht auf die bisher als Bierkontaminanten beschriebenen Mikroorganismen beschränkt. Vielmehr bietet der Einsatz der erfindungsgemäßen Nukleinsäuremoleküle und Verfahren die Möglichkeit, das Vorhandensein von anderen brauereirelevanten Mikroorganismen zu erkennen, die bisher nicht als Bierkontaminanten beschrieben wurden. Ein positives Ergebnis auf der Ebene höherer taxonomischer Einheiten (z.B. Ordnungen, Familien, Gattungen) verbunden mit einem negativen Ergebnis auf der Ebene der bekanntermaßen brauereire;evanten niederen taxonomischen Einheiten (z.B. Spezies, Subspezies, Stämmen) zeigt eine Kontamination mit einem solchen untypischen brauereirelevanten Mikroorganismus an.

In einem ersten Schritt des erfindungsgemäßen Verfahrens wird die zu untersuchende Probe mit einer Kombination aus mindestens zwei ersten Nukleinsäuremolekülen (Primem) in Kontakt gebracht. Diese Nukleinsäuremoleküle hybridisieren mit einem Bereich einer mikrobiellen Nukleinsäure, der in brauereirelevanten Mikroorganismen konserviert ist. Die Hybridisierung erfolgt durch Paarung der Primer mit Bereichen der mikrobiellen Nukleinsäure, die eine zumindest teilweise komplementäre Basensequenz aufweisen. Durch den Begriff "konserviert" wird die evolutionäre Variabilität von Nukleotidsequenzen für Spezies verschiedener taxonomischer Einheiten charakterisiert. Vergleicht man sich entsprechende Sequenzabschnitte von mindestens zwei beliebigen brauereirelevanten Mikroorganismen, so kann die Sequenz als variabel oder als konserviert angesehen werden. Als konserviert werden solche Vergleichsequenzen bezeichnet, die zu mindestens 95%, als variabel solche die weniger als 95% identisch sind. Ein in brauereirelevanten Mikroorganismen konservierter Bereich einer Nukleinsäure bezeichnet also einen Bereich, der in allen brauereirelevanten Mikroorganismen (wie oben definiert) zu mindestens 95% identisch ist.

In einer bevorzugten Ausführungsform der vorliegenden Erfindung kommt der konservierte Bereich in einem Genomabschnitt vor, der die bakteriellen 23S- und 5S-Gene enthält Dieser Bereich umfaßt den intergenischen Spacer zwischen den Genen für die 23S-rRNA und die 5S-rRNA sowie die angrenzenden 23S- und 55-rDNA-Gene und schließt sowohl konservierte Sequenzbereiche als auch hypervariable (d.h. sehr keimspezifische) Sequenzbereiche ein. Prokaryontische Ribosomen beinhalten in der Regel drei distinkte Nukfelns4urekomponenten, welche allgemein als 5S, 16S und 23S rRNA (ribosomale Nukteinsäune) bekannt sind. Die genetische Information für diese Ribonukleinsäuren (rDNA) ist im Genom typischerweise als Tandem angeordnet. Die typische Organisation einer solchen Einheit ist 16S-23S-5S, wobei die Gene über kurze hypervariable intergenische Bereiche, sogenannte Spacer, miteinander verbunden sind. Die Einheiten sind im Genom mehrfach vorhanden, wobei die Anzahl der Operons von Art zu Art variieren kann. Die hohe Konservierung der DNA-Sequenz in bestimmten Abschnitten der ribosomalen DNA über das gesamte Bakterienreich ermöglicht ein Design von nicht spezifischen Oligonukleotiden auch ohne genaue Kenntnis der einzelnen DNA-Sequenzen der zu untersuchenden Organismen. Die erfindungsgemäßen Sequenzen nach SEQ ID NO 1-20 (fabelte 1) stellen Sequenzen des 23S-5S intergenischen Spacers sowie der augmuzenten 23S und 55S-DNA Gene von brauereirelevanten Mikroorganismen dar, von denen Nukleinsäuremoleküle zum Einsatz in das erfindungsgemäße Verfahren abgeleitet werden können.

Die im ersten Schritt des erfindungsgemäßen Verfahrens eingesetzte Kombination aus mindestens zwei ersten Nukleinsäuremolekülen wird so gewählt daß sie als Primer in ' einer Amplifkationsreaktion einsetzbar sind, d.h. ein Nukleinsäuremolekül hybridisiert an einen ersten konservierten Bereich des ersten Stranges der Ziel-DANN und das andere Nukleinsäuremolekül an einen.zweiten konservierten Bereich des zum ersten komplementären DNA-Stranges, wobei der gewünschte Zielbereich der DNA eingeschlossen wird. Beide Nukleinsäuremoleküle weisen eine Länge von mindestens 10 bp, vorzugsweise 15-30 bp auf. In einer bevorzugten Ausführungsform der Erfindung wird eine Kombination aus mindestens zwei Nukleinsäuremolekülen gemäß dieser Erfindung eingesetzt. In einer besonders bevorzugten Ausführungsform der Erfindung wird eine Kombination eingesetzt, die mindestens ein Nukieinsäurernotekül mit einer Sequenz nach einer der SEQ ID NO 48-54 (Tabelle 2) umfaßt.

in einem zweiten Schritt des erfindungsgemäßen Verfahrens wird die mikrobielle Nukleinsäure oder ein Teil derselben amplifiziert, wodurch mindestens ein Amplifikationsfragment erzeugt wird. Unter Amplifikation wird die Erhöhung der Konzentration einer in einem Reaktionsgemisch vorliegenden Nukleinsäure oder eines Teils derselben verstanden. Eingesetzte Verfahren zur Amplifikation von Nukleinsäuren sind z.B. die PCR (US 4 683 195, US 4 683 202, US 4 965188), die "self-sustained sequence replication" (EP 329 822), das "transcription based amplification System" (EP 310 229) und das "β-RNA-Replicase-System" (US 4 957 858). In einer bevorzugten Ausführungsform der Erfindung umfaßt das Amplifizieren eine Polymerase-Kettenreaktion (PCR). In einer weiteren Ausführungsform der vorliegenden Erfindung umfaßt das Amplifizieren eine Ligasen-Kettenreaktion oder eine isotherme Nukleinsäureamplifikation.

In einem dritten Schritt des Verfahrens gemäß der vorliegenden Erfindung werden die erhaltenen Amplifikationsfragmente mit mindestens einem zweiten Nukleinsäuremolekül (Sonde) in Kontakt gebracht Dieses Nukleinsäuremolekül bzw. diese Nukleinsäuremoleküle hybridisieren spezifisch mit mindestens einem Amplifikationsfragment, das eine Sequenz der mikrobiellen Nukleinsäure umfaßt die für L. brevis spezifisch ist, d.h. nur in Mitgliedem dieser Art vorkommt.

Als Hybridiserung wird die Doppelstrangbildung zweier identischer oder ähnlicher Nukleobctfragmente (DNA, RNA, PNA) bezeichnet Von spezifischer Hybridisierung spricht man, wenn eine stabile Hybridnukleinsäure zwischen dem Oligonukleotid und der entsprechenden Ziel-DNA des Oligonukleotides besteht, nicht jedoch zu anderer DNA als der Ziel-DNA. Im Sinne dieser Erfindung weist das Merkmal "Sequenz, die mit einer Sequenz nach (i) spezifisch hybridisiert" auf eine Sequenz hin, die unterstringenten Bedingungen mit der Sequenz nach (i) hybridisiert. Beispielsweise können die Hybridisierungen bei 50°C mit einer Hybridisierungslösung bestehend aus 2,5 x SSC, 2 x Denhardts Lösung, 10 mM Tris, 1 mM EDTA pH 7,5 durchgeführt werden. Als Waschbedingungen eignen sich z.B. viermal wiederholte 1 minütige Waschungen in 0.1 x SSC bis 1,0 x SSC, 2 x Denhardts, 10 mM Tris, 1 mM EDTA, pH 7,6 bei 20-50°C.

In einer bevorzugten Ausführungsform der Erfindung wird als zweites Nukleinsäuremotekül (Sonde) ein oder mehrere der erfindungsgemäßen Nukleinsäuremoleküle eingesetzt.

Als speziesspezfische Nukleinsäuresonden werden Nukleinsäuremoleküle verstanden, die mit der DNA von allen lsolaten der jeweiligen nachzuweisenden Spezies unter denselben Stringenzbedingungen hybridisieren. Erfindungsgemäße, für L. brevis speziesspezifische Nukleinsäuremoleküle mit den SEQ ID NO 21, SEQ ID NO 73-74. (Tabelle 2) können eingesetzt werden.

Im letzten Schritt des erfindungsgemäßen Verfahrens erfolgt der Nachweis von mindestens einer Hybridnukleinsäure, die aus einem Amptifikationsfragment und einem im vorangegangenen Schritt eingebrachten zweiten Nukleinsäuremolekül besteht

Vorzugsweise sind zweite Nukleinsäuremoleküle (Sonde) 15-30 Nukleotide lang. In einer Ausführungsform der vorliegenden erfindung sind die zweiten Nukleinsäuremoleküle dadurch modifiziert, daß bis zu 20% der Nukleotide in 10 aufeinanderfolgenden Nukleotiden, insbesondere 1 oder 2 Nukleotide aus einem 10er-Block durch Nukleotide ersetzt sind, die in Bakterien nicht natürlich vorkommen.

Das erfindungsgemäße Verfahren umfaßt vorzugsweise die sogenannte Consensus-PCR. Bei dieser Methode erfolgt eine Vervielfältigung der mikrobiellen Nukleinsäure oder eines Teils derselben und eine anschließende Detektion dieser Moleküle mittels Hybridisierung mit markierten spezifischen Sonden. In der Consensus-PCR werden Nukleinsäuremoleküle eingesetzt, die es ermöglichen, von mehreren oder gar allen der relevanten Stämme, Subspezies, Spezies oder Gattungen ein Amplifikationsprodukt zu erhalten. Die Amplifikationsreaktion führt nicht zu einer Differenzierung der Mikroorganismen. Die Spezifität des Nachweises wird durch die anschließende Hybridisierungsreaktion mit spezifischen Sonden erreicht Auf diese Art und Weise können brauereirelevante Mikroorganismen simultan in einer einfachen Kombination aus Amplifikations- und Detektionsreaktion erfaßt werden.

Diese Art der Amplifikation und Detektion ermöglicht die Automatisierbarkeit der Detektionsreaktion, so daß ein hoher Probendurchsatz ermöglicht wird. Es kann beispielsweise ein PCR-ELISA-Nachweisverfahren eingesetzt werden, bei dem die entsprechenden Sonden in verschiedene Kavitäten einer Mikrotiterplatte gebunden werden, in denen anschließend die Hybridisierung und der Nachweis der markierten Amplifikate geschieht. Der Nachweis kann auch durch die Verwendung eines Microarrays erfolgen, auf dem mehrere Sonden immobilisiert sind, wodurch die Nachweisreaktion schnell und ohne großen Aufwand durchgeführt werden kann.

In einer bevorzugten Ausführungsform der Erfindung ist das zweite Nukleinsäuremolekül (Sonde) so modifiziert bzw. markiert, daß es ein nachweisbares Signal erzeugen kann. Die Modifikation bzw. Markierung wird ausgewählt aus (i) radioaktiven Gruppen (ii) farbigen Gruppen, (iii) fluoreszierenden Gruppen, (iv) Gruppen zur Immobilisierung an einer festen Phase und (v) Gruppen, die eine indirekte oder direkte Reaktion, insbesondere mit Hilfe von Antikörpem, Antigenen, Enzymen und/oder Substanzen mit Affinität zu Enzymen oder Enzymkomplexen erlauben.

Als Markierung werden im Sinne dieser Erfindung direkt oder indirekt nachweisbare Gruppen oder Gruppen zur Immobilisierung an eine feste Phase bezeichnet, die an das Nukleinsäuremolekül angehängt sind. Direkt nachweisbar sind Metallatome, radioaktive, farbige oder fluoreszierende Gruppen. Indirekt nachweisbar sind immunologisch oder enzymatisch nachweisbare Gruppen, wie z.B. Antigene und Antikörper, Haptene oder Enzyme oder enzymatisch wirkende Teile von Enzymen. Diese indirekten Gruppen werden in nachfolgenden Reaktionen nachgewiesen. Bevorzugt sind Haptene, die an ein Oligonukleotid gekoppelt sind und die man in einer anschließenden Antikörperreaktion nachweist.

Die erfindungsgemäßen Nukleinsäuremoleküle können zum Nachweis und/oder zum Identifizieren und/oder Charakterisieren von brauereirelevanten Bakterien verwendet werden. Verwendung können die hier beschriebenen Primer und/oder Sonden auch bei der Detektion der beschriebenen Keime in anderen Getränken als Bier, in anderen Proben aus dem Brauereibereich, wie z.B. in Rohstoffen, Anstellhefe, Umgebungsproben, in anderen Lebensmittelproben oder in klinischen Proben etc. finden.

### Beispiele:

### Beispiel 1: Bestimmung der DNA-Zielsequenz der bierschädlichen Bakterien und nahverwandter Arten

Durch Sequenzvergleich bekannter 23S-rDNA und 5S-rDNA Sequenzen (GenBank Sequence Database des National Center of Biotechnology Information: NCBI) wurden konservierte Genbereiche ermittelt, die als Hybridisierungsorte der zur Sequenzierung verwendeten Primer dienen. Aus Reinkulturen der in Tabelle 1 aufgeführten Bakterien wurde durch an sich bekannten Standardverfahren genomische DNA isoliert. Mit Primem, die in hochkonservierten Bereichen hybridisieren, wurden in einer PCR von allen nachzuweisenden Bakterien Amplifikate erhalten. Folgende Primer wurden für die Amplifikation und die anschließende Sequenzierung verwendet:
Primer 1 = SEQ ID NO 47:
   5'-AAG TGC TGA AAG CAT CTA AG-3'
Primer 2 = SEQ ID NO 55:
   5'-GGC RRY GTC TAY TYT CSC-3'

Zusammensetzung der PCR:

| | | |
|---|---|---|
| Genomische DNA (10-100 ng) | 1,00 µl | |
| H₂O | 16,85 µl | |
| Puffer (10 x) | 2,50 µl | 1 x |
| dNTP (10 mM) | 0,50 µl | 200 µM |
| Primer 1=Seq ID NO 48 (5 µM) | 1,50 µl | 0,30 µM |
| Primer 2=Seq ID NO 49 (5 µM) | 1,50 µl | 0,30 µM |
| MgCl₂ (50 mM) | 1,00 µl | 2,00 mM |
| Taq-Polymerase (5 U/µl) | 0,15 µl | 0,03 U/µl |
| Σ | 25,00 µl | |

### Temperaturprofil:

| | | |
|---|---|---|
| 5' | 95°C | |
| 30" | 95°C | |
| 30" | 50°C | x38 |
| 30" | 72°C | |
| 5' | 72°C | |

Diese Amplifikate wurden über ein Agarosegel und durch eine anschließende Behandlung mit dem QIAquick PCR Gel Extraction Kit (Fa.Quiagen) gereinigt und mit den o.g. Primem, die mit einer IRD-800 Markierung versehen sind, in dem Long Read Sequencer Modell 4000L (Fa.LI-COR) sequenziert. Die resultierenden Sequenzen der 23S/5S-rDNA-Spacerbereiche der brauereirelevanten Bakterien und der phylogenetisch nah verwandten Arten wurden miteinander verglichen und Sequenzbereiche bestimmt, die
1.) in allen Spezies der jeweiligen nachzuweisenden Gattung zu finden sind und sich gleichzeitig von denen anderer Gattungen bzw. Spezies unterscheiden.
2.) nur in der jeweiligen zu bestimmenden Spezies zu finden sind, sich jedoch von anderen zu erfassenden und nicht zu erfassenden Bakterien unterscheiden.

In den unter 1.) beschriebenen Sequenzbereichen wurden Hybridisierungsstellen gattungsspezifischer Oligonukleotide, in den unter 2.) beschriebenen Sequenzbereichen die Bindungsstellen speziesspezifischer Oligonukleotide definiert.

### Beispiel 2: Nachweis von bierschädlichen Bakterien mit der Polymerase-Kettenreaktion

### I. Amplifikation

Aus Reinkulturen der in Tabelle 1 aufgeführten Bakterien wurde in an sich bekannten Standardverfahren genomische DNA isoliert. Dekadische Verdünnungen von 1 fg/µl bis 1 pg/µl dieser Präparationen wurden anschließend in eine PCR mit folgender Zusammensetzung eingesetzt:
Primer 3 = SEQ ID NO 46:
   5'-AAG GGC CAT CRC TCA ACG G -3'
Primer 4 = SEQ ID NO 48:
   5'-TGT GTT CGi iAT GGG AAC AGG TG -3'

| | | | |
|---|---|---|---|
| Genomische DNA | 1,00 µl | 4,00 µl | |
| H₂O | 16,60 µl | 66,40 µl | |
| Puffer (10x) | 2,50 µl | 10,00 µl | 1x |
| dNTP (10 mM) | 0,50 µl | 2,00 µl | 0,20 mM |
| Primer 3= SEQ ID NO 21 (5 µM) | 1,50 µl | 6,00 µl | 0,30 mM |
| Primer 4= SEQ ID NO 22 (5 µM) | 1,50 µl | 6,00 µl | 0,30 mM |
| Digoxigenin markiert | | | |
| DMSO (100%) | 0,25 µl | 1,00 µl | 1,00 % |
| MgCl₂ (50 mM) | 1,00 µl | 4,00 µl | 2,00 mM |
| Taq-Polymerase (5U/µl) | 0,15 µl | 0,60 µl | 0,03 U/µl |
| Σ | 25,00 µl | 100,00 µl | |

Die PCR wurde unter folgenden Bedingungen in dem Mastercycler® gradient (Fa.EPPENDORF) nach folgendem Temperaturprofil durchgeführt:

| | | |
|---|---|---|
| 5' | 95°C | |
| 30" | 95°C | |
| 45" | 55°C | x38 |
| 90" | 72°C | |
| 5' | 72°C | |

Primer 3 (SEQ ID NO 46) wurde durch Sequenzvergleich bekannter 23S-rDNA Sequenzen (GenBank Sequence Database des NCBI) bestimmt. Er hybridisiert an hochkonservierte Sequenzabschnitte im 23S-rDNA Genbereich. Der Bindungsort liegt außerhalb des mit den Primern SEQ ID NO 48, 49 sequenzierten Bereiches.

Primer 4 (SEQ ID NO 48) wurde aufgrund eigener Sequenzdaten bestimmt. Der Hybridisierungsort von Primer 2 liegt angrenzend an dem intergenischen 23S/5S-Spacer im 5S-rRNA Genbereich.

### II. Detektion im PCR-ELISA

Die Detektion erfolgt im PCR-ELISA. Dazu werden je verwendeter Sonde 5 µl Amplifikat mit 5 µl Denaturierungspuffer (125 mM NaOH, 20 mM EDTA, pH 14) versetzt und 15 min. bei Raumtemperatur inkubiert. Jeweils 2 pmol der jeweiligen biotinylierten Sonde werden in 100 µl Hybridisierungspuffer (2,5xSSC, 2x Denhardts Lösung, 10 mM Tris, 1 mM EDTA pH 7,5) pipettiert und in die Kavitäten einer mit Streptavidin beschichteten Mikrotiterplatte überführt und bei der Hybridisierungstemperatur von 50°C vorinkubiert. Nach der Denaturierung wird der Denaturierungsansatz zu dem Hybridisierungsansatz pipettiert. Anschließend erfolgt die 30 minütige Inkubation bei Hybridisierungstemperatur. Ist die Hybridisierung abgeschlossen, wird der Hybridisierungsansatz entfernt und 4x mit 200 µl Waschpuffer 1 (WP1: 0,1 x SSC, 2 x Denhardts, 10 mM Tris, 1 mM EDTA, pH 7,6) jeweils für 1 min. bei Hybridisierungstemperatur gewaschen. Anschließend werden 100 µl einer nach Herstellerangaben verdünnten Lösung eines mit einer Meerrettichperoxidase konjugierten Anti-Digoxigenin-Antikörpers zugegeben (Boehringer Mannheim). Das Konjugat wird in Waschpuffer 2 (WP2: 100 mM Tris, 150 mM NaCl, 0,05% Tween 20, 0,5% Blocking-Reagenz, 100 µg/ml Heringssperma, pH 7,6) verdünnt. Anschließend erfolgt die Antikörper-Inkubation bei 37°C für 30 min.. Danach wird viermal mit 200 µl WP 2 gewaschen (bei Raumtemperatur). Nach dem Waschen werden 100 µl POD-Substrat (Boehringer Mannheim) zugegeben und 20 min. bei RT inkubiert. Anschließend wird die Farbreaktion mit 100 µl 0,5 M H₂SO₄ abgestoppt und bei 450 nm gemessen.

### III. Auswertung

Gemäß dem oben aufgeführten Detektionsprotokoll wurde der Nachweis für alle untersuchten Bakterien und Bakteriengruppen unter Verwendung der entsprechenden gattungs- und speziesspezifischen Sonden geführt. Gattungsspezifische Sonden sind für *Pediococcus* SEQ ID NO 35, für *Pectinatus* SEQ ID NO 36, für *Megasphaera* SEQ ID NO 37, für *Selenomonas* SEQ ID NO 38, für *Zymophilus* SEQ ID NO 39. Speziesspezifische Sonden sind für *Lactobacillus brevis* SEQ 10 NO 21, *Lactobacillus lindneri* SEQ ID NO 22, *Lactobacillus casei* + *Lactobacillus paracasei* SEQ ID NO 23, *Lactobacillus coryniformis* SEQ 10 NO 24, *Lactobacillus curvatus* SEQ ID NO 25, *Pediococcus damnosus* SEQ ID NO 26, *Pediococcus inopinatus* SEO ID NO 27, *Pectinatus cerevisiiphilus* SEQ ID NO 28, *Pectinatus frisingiensis* SEQ 10 NO 29, *Pectinatus sp.* DSM20764 SEQ ID NO 30, *Megasphaera cerevisiae* SEQ ID NO 31, *Selenomonas lacticifex* SEQ ID NO 32, *Zymophilus paucivorans* SEQ ID NO 33 und *Zymophilus raffinosivorans* SEQ ID NO 34.

Als Kontrolle wurden die Consensus-Sonden SEQ ID NO 40 und 41 eingesetzt, die an die Amplifikate sämtlicher nachzuweisender Spezies hybridisieren. Weitere mögliche Bindungsstellen für Consensus-Sonden sind SEQ ID NO 42-45. Die Sonden der SEQ ID NO 40 bis 45 sind durch Sequenzvergleich bekannter 23S-rDNA und 5S-rDNA Sequenzen (GenBank Sequence Database, NCBI) ermittelt worden.

War die gemessene Extinktion bei einer eingesetzten Menge genomischer DNA von 10 fg in die PCR größer als 1, so wurde das Ergebnis positiv bewertet. Die Ergebnisse des PCR-ELISA sind in Tabelle 3 dargestellt.

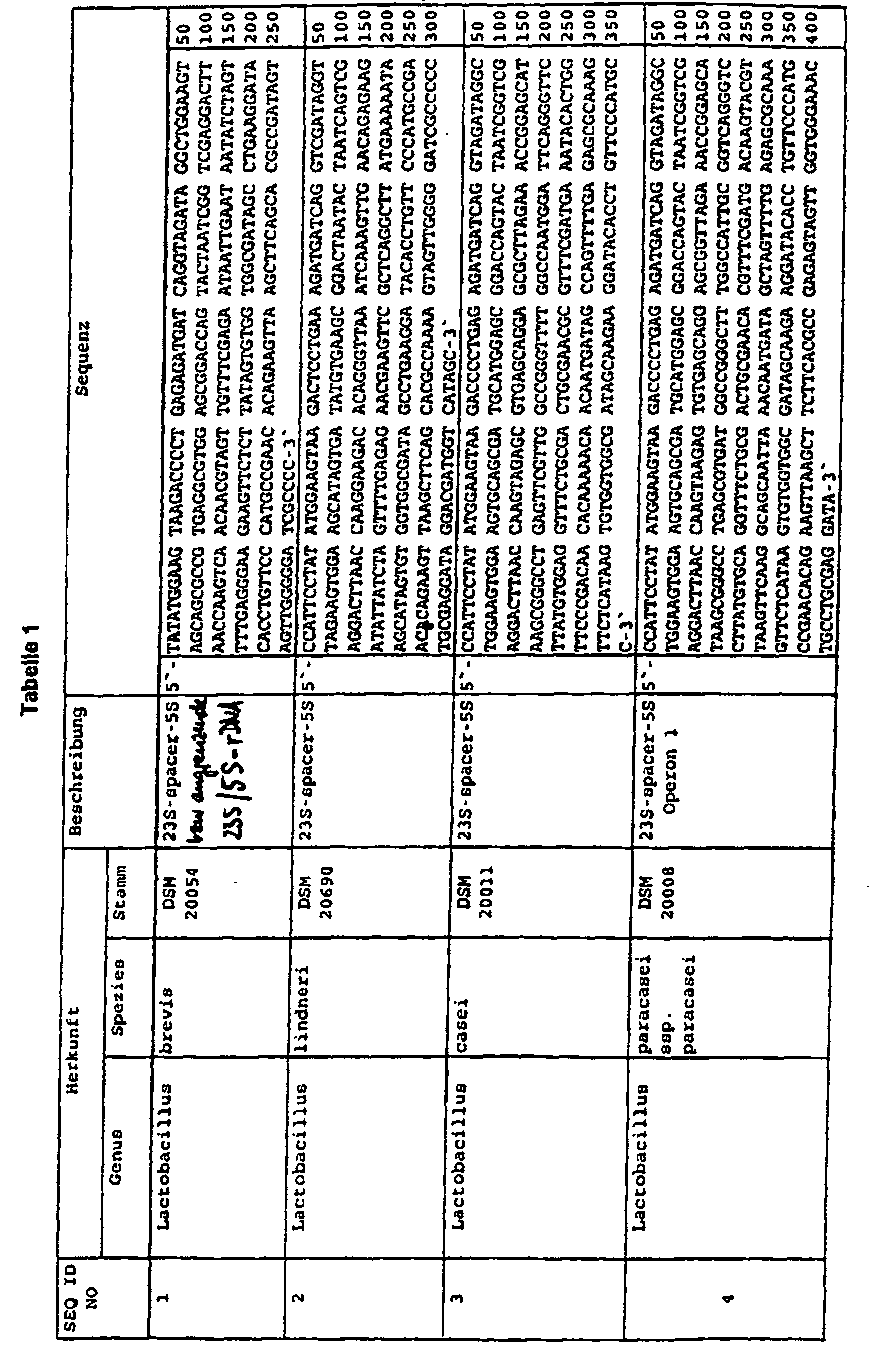

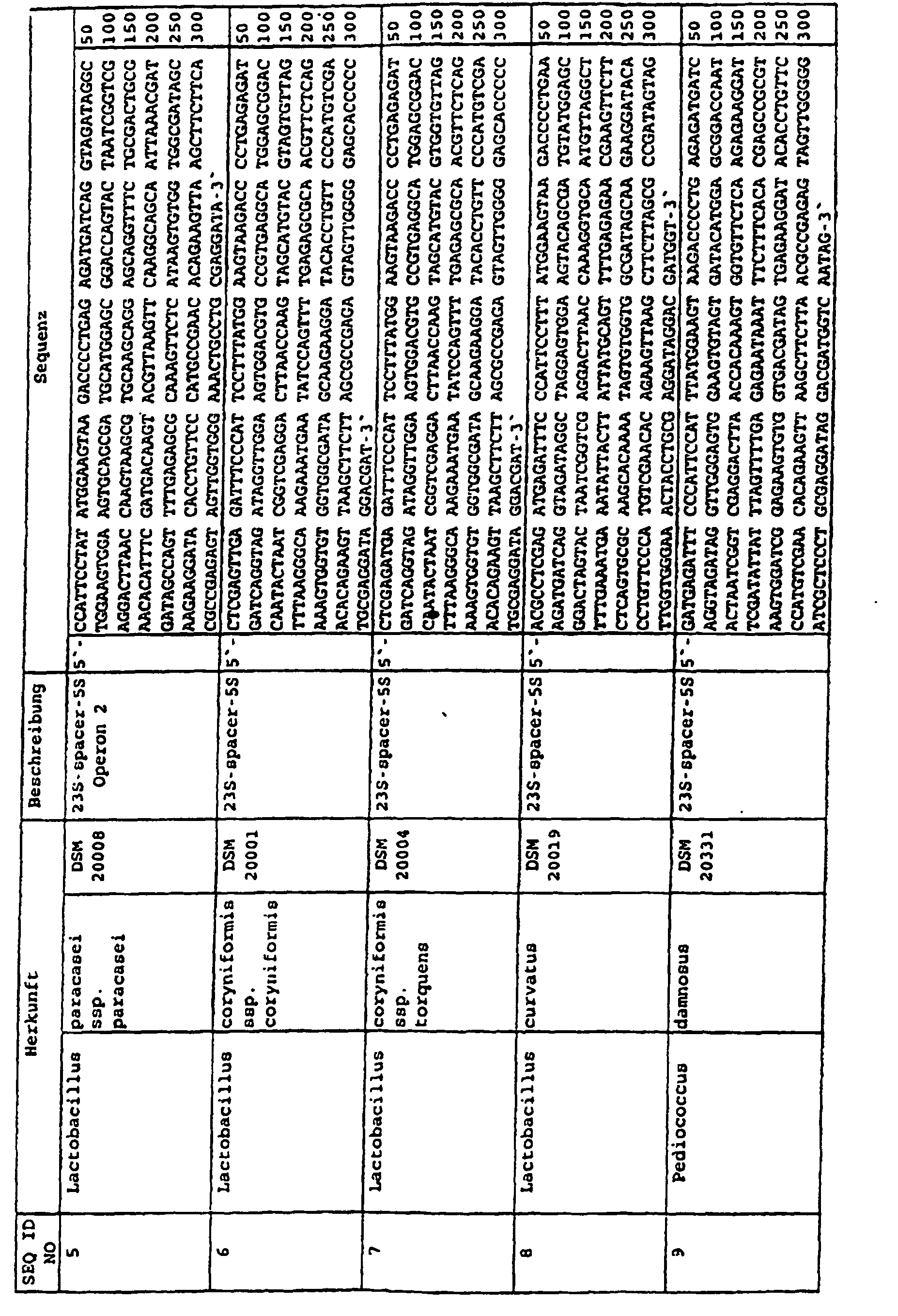

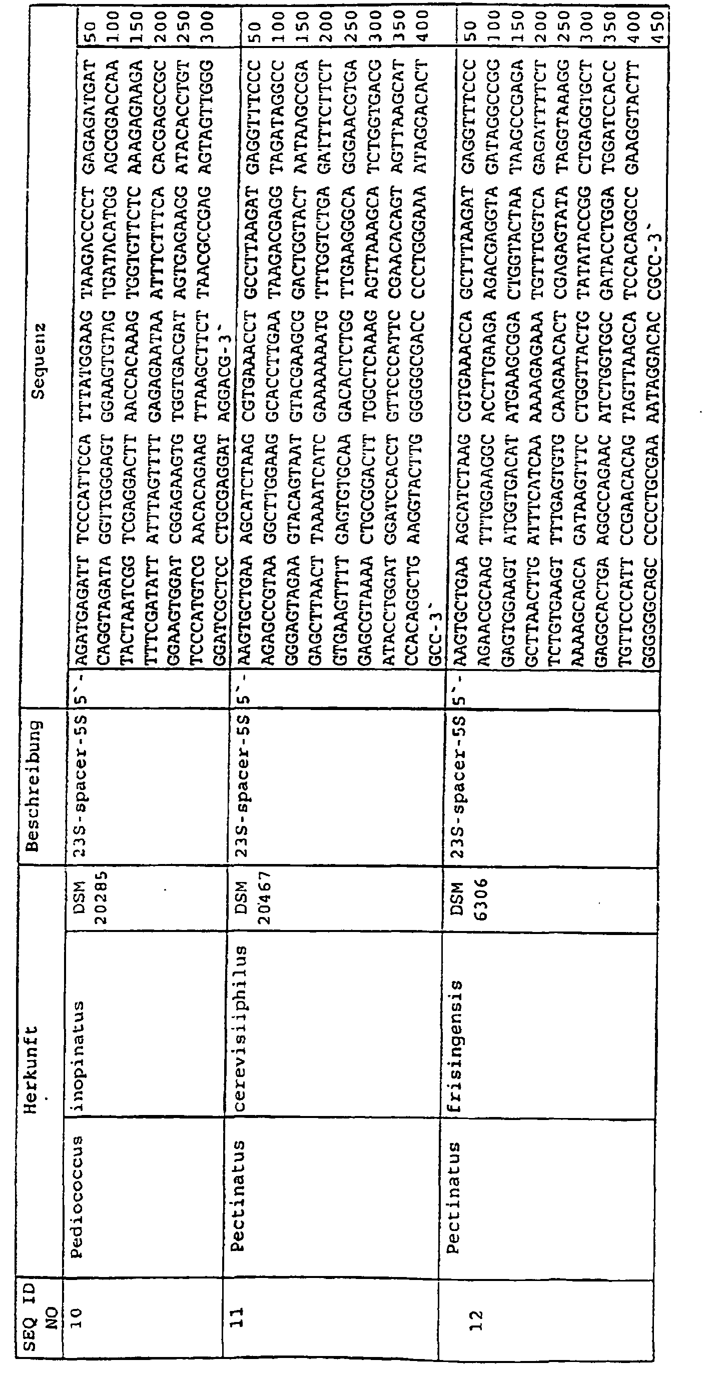

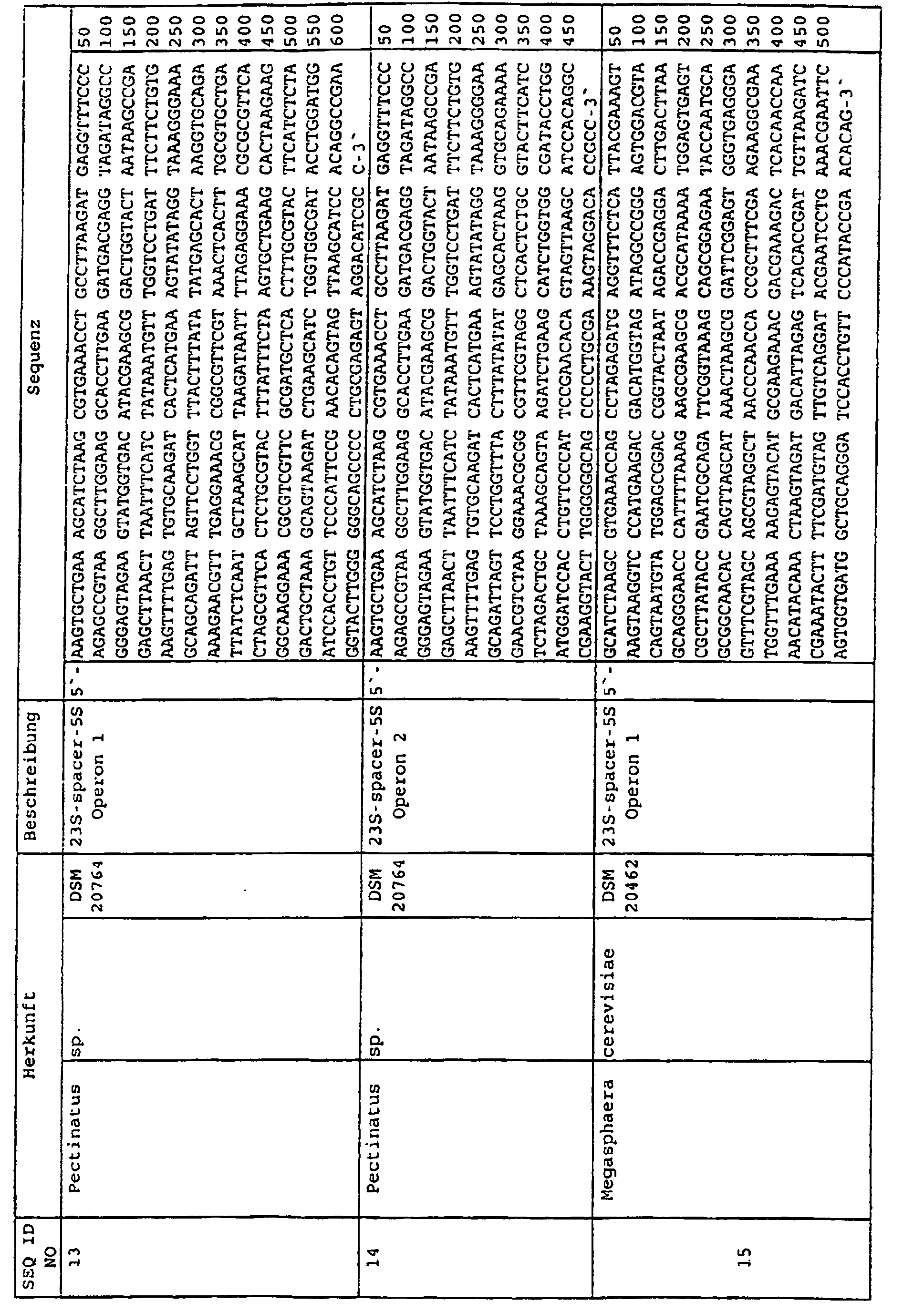

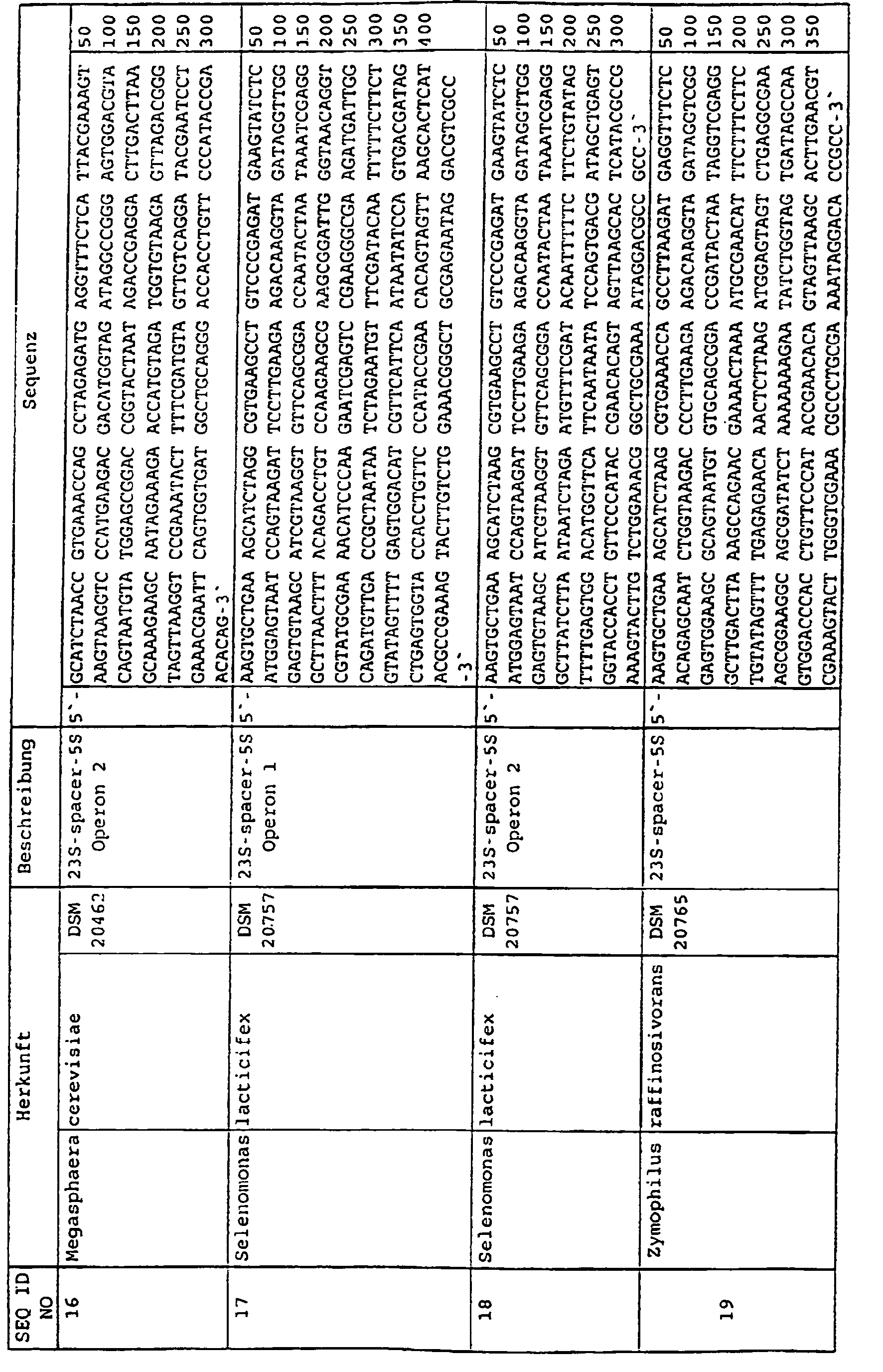

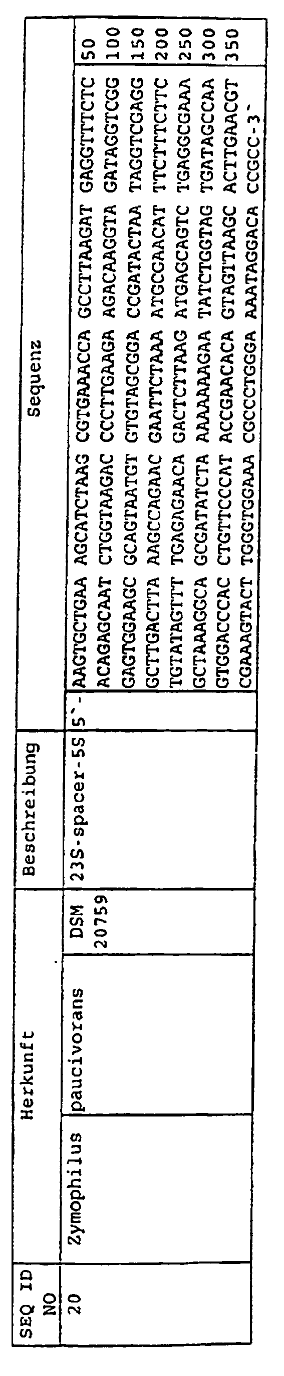

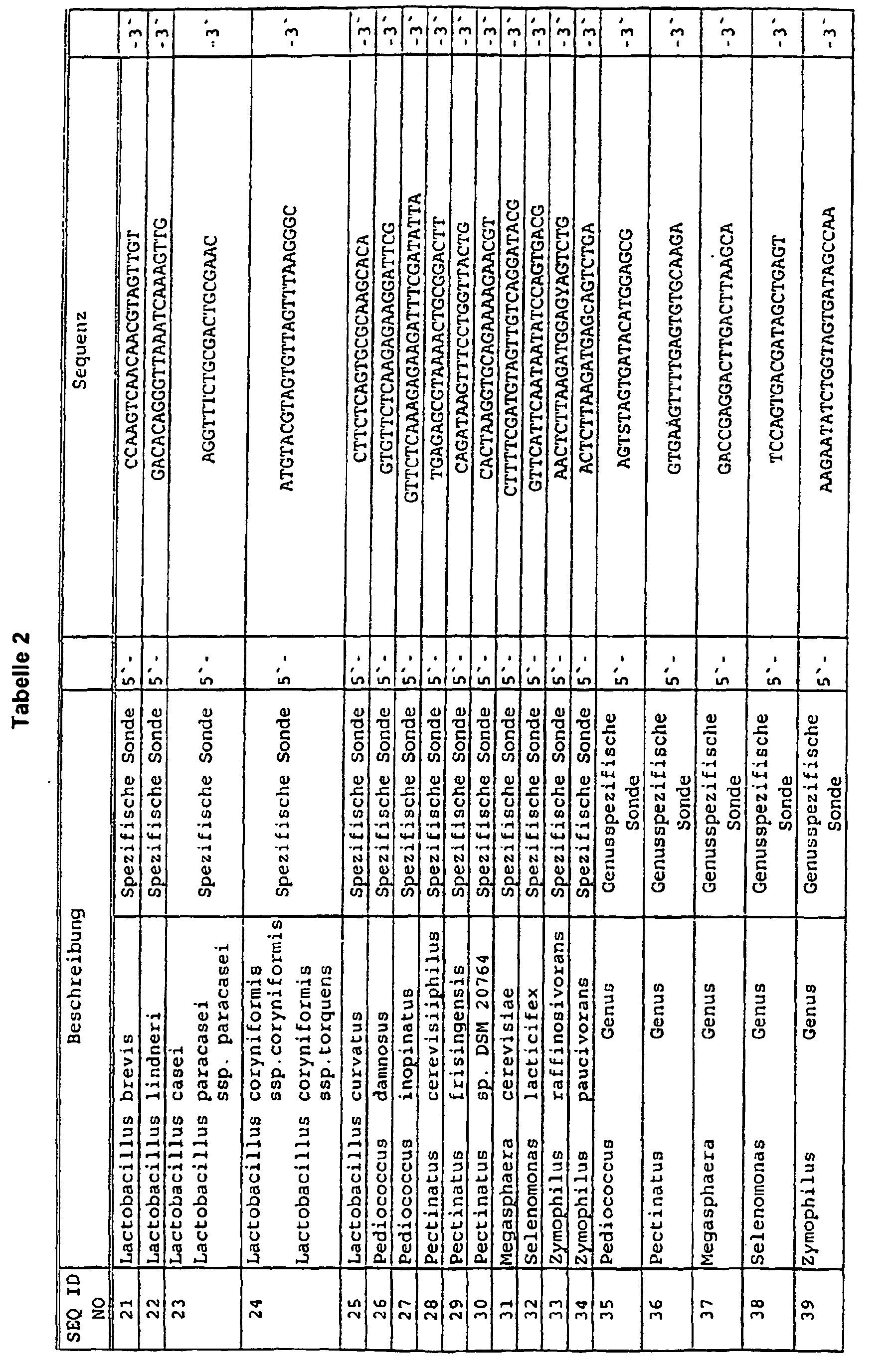

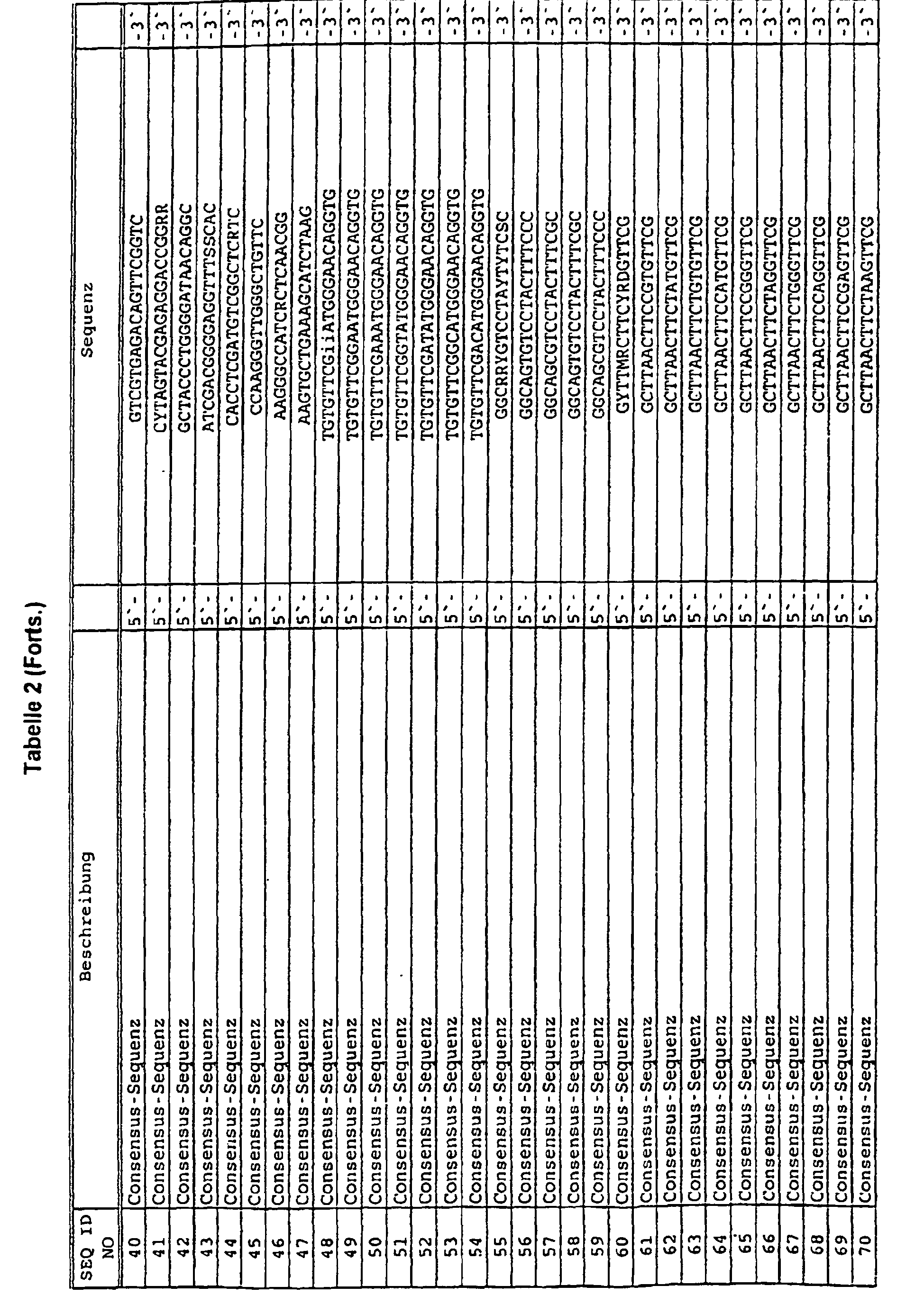

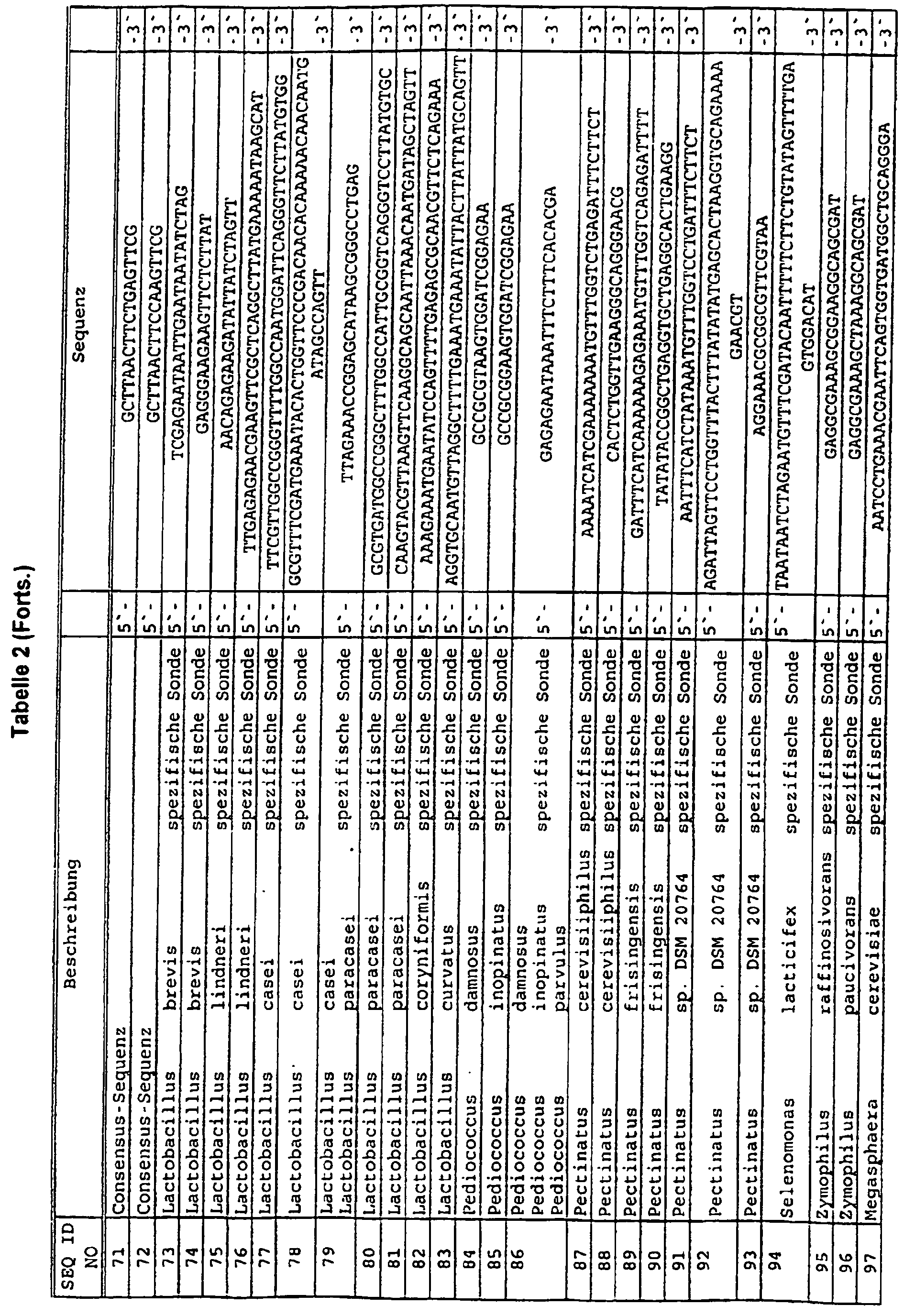

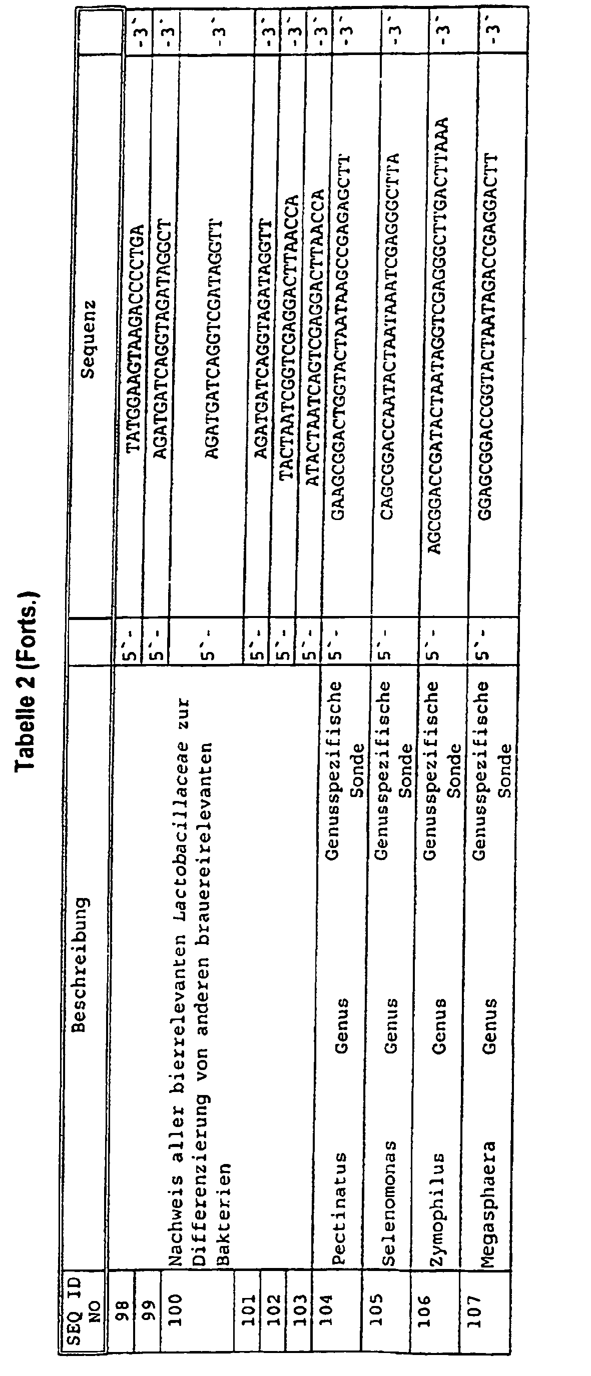

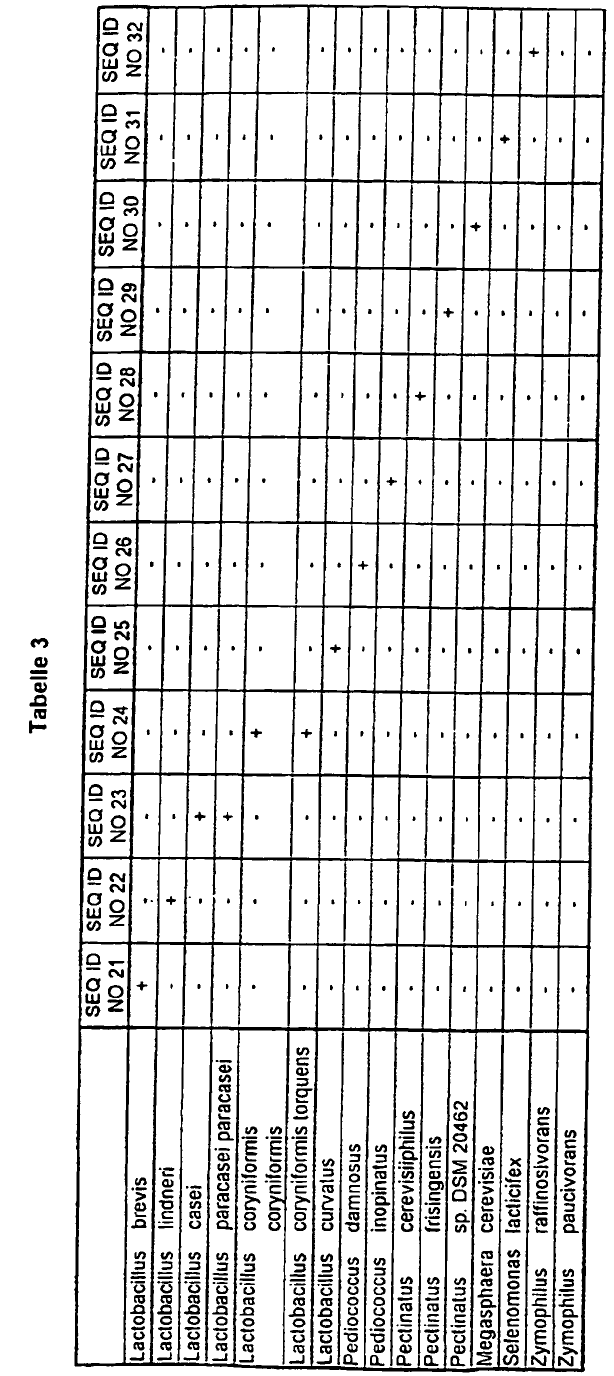

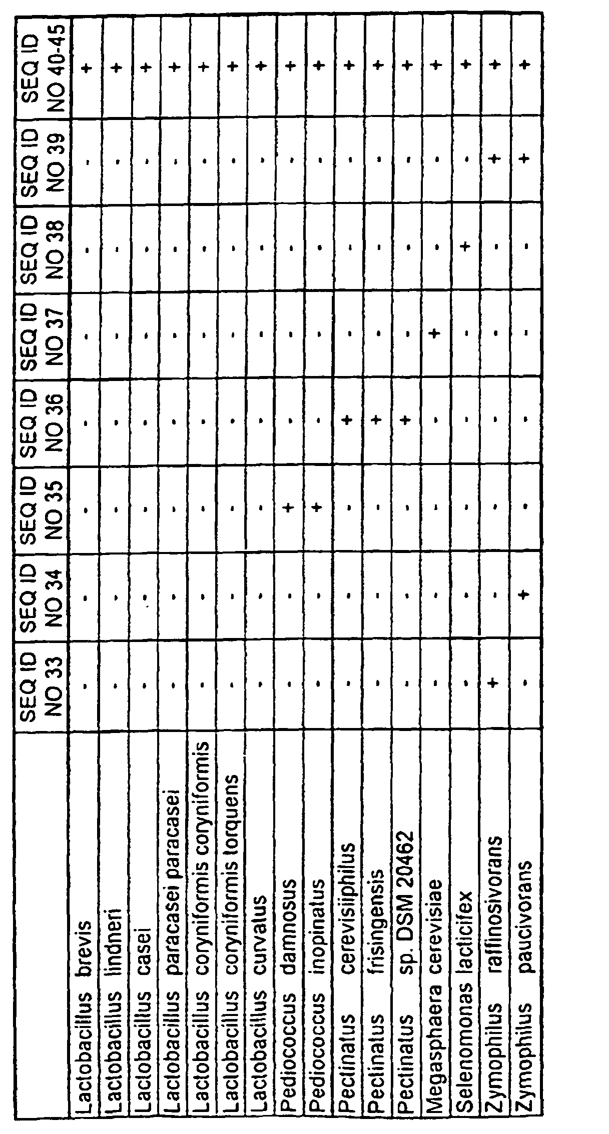

## Patentansprüche

1. Verfahren zum Nachweis von *Lactobacillus brevis* in einer Probe, das folgende Schritte umfasst:
(a) Inkontaktbringen der Probe mit einer Kombination aus mindestens zwei ersten Nukleinsäuremotekülen (Primem), die mit einem in allen Bakterien der Spezies lactobacillus brevis, Lactobacillus lindneri, lactobacillus casei, Lactobacillus paracasei, Lactobacillus coryniformis, Lactobacillus curvatus, Pediococcus damnosus, Pediococcus inopinafus, Pectinatus cerevisiiphilus, Pectinatus frisingiensis, Pectinatus sp. DSM 20764. Megasphaera cerevisiae, Selenomonas lacticifex, Zymophilus paucivorans, Zymophilus raffinosivorans konservierten Bereich einer mikrobiellen Nukleinsäure hybridisieren;
wobei die mindestens 2 ersten Nukleinsäuremoleküle vom intergenischen Spacer 23S-5S bzw. den angrenzenden 23S- oder 5S-rDNA-Genregionen aus L. brevis abgeleitet, d.h. wie in SEQ ID NO:1. dargestellt sind;
(b) Amplifizieren der mikrobiellen Nukleinsäure oder eines Teils derselben zur Erzeugung von mindestens einem Amplifikationsfragment;
(c) Inkontaktbringen der in Schritt (b) erhaltenen Amplifikationsfragmente mit mindestens einem zweiten Nukleinsäuremolekül (Sonde), welches mit mindestens einem Amplifikationsfragment spezifisch hybridisiert, das eine für Bakterien der Art *Lactobacillus brevis* spezifische Sequenz der mikrobiellen Nukleinsäure umfasst,
wobei das mindestens eine zweite Nukleinsäuremolekül vom intergenischen Spacer 23S-5S bzw. den angrenzenden 23S- oder 5S-rDNA Genregionen aus L. brevis abgeleitet, d.h. wie in SEQ ID NO:1 dargestellt, ist;
(d) Nachweis von mindestens einer Hybridnukleinsäure, die aus einem Amplifikationsfragment und einem im Schritt (c) eingebrachten zweiten Nukleinsäuremolekül besteht.

2. Verfahren nach Anspruch 1, **dadurch gekennzeichnet, dass** das Amplifizieren eine Polymerase-Kettenreaktion (PCR) umfässt.

3. Verfahren nach Anspruch 1, **dadurch gekennzeichnet, dass** das Amplifizieren eine Ligasen-Kettenreaktion umfasst.

4. Verfahren nach Anspruch 1, **dadurch gekennzeichnet, dass** das Amplifizieren eine isotherme Nukleinsäureamplifikation umfasst.

5. Verfahren nach einem der Ansprüche 1 bis 4, **dadurch gekennzeichnet, dass** das zweite Nukleinsäuremotekül zur Erzeugung eines nachweisbaren Signals modifiziert bzw. markiert ist, wobei die Modifikation bzw. Markierung ausgewählt wird aus (i) radioaktiven Gruppen (ii) farbigen Gruppen, (iii) fluoreszierenden Gruppen, (iv) Gruppen zur Immobilisierung an einer festen Phase und (v) Gruppen, die eine indirekte oder direkte Reaktion, insbesondere mit Hilfe von Antikörpem, Antigenen, Enzymen und/oder Substanzen mit Affinität zu Enzymen oder Enzymkomplexen erlauben.

6. Verfahren nach einem der vorangegangenen Ansprüche, **dadurch gekennzeichnet, dass** die ersten Nukleinsäuremoleküle und/oder das zweite Nukleinsäuremolekül mindestens 10 Nukleotide, vorzugsweise 15-30 Nukleotide lang sind.

7. Verfahren nach einem der vorangegangenen Ansprüche, **dadurch gekennzeichnet, dass** die ersten Nukleinsäuremoleküle und/oder das zweite Nukleinsäuremolekül **dadurch** modifiziert sind, dass bis zu 20% der Nukleotide in 10 aufeinanderfolgenden Nukleotiden, insbesondere 1 oder 2 Nukleotide aus dem 10er-Block durch Nukleotide ersetzt sind, die in Bakterien nicht natürlich vorkommen.

8. Verfahren nach einem der vorangegangenen Ansprüche, **dadurch gekennzeichnet, dass** der konservierte Bereich in dem Genomabschnitt vorkommt, der 23 S- und 5 S-Gene aus Bakterien der Art *Lactobacillus brevis* enthält.

9. Nukeinsäuremolekül geeignet ats Sonde zum keimspezifischen Nachweis von Baicterien der Art *Lactobacillus brevis,* ausgewählt aus:
(i) einer Nukleinsäure mit einer Sequenz nach SEQ ID NO 21 oder SEQ 10 NO 73-74 oder einem 15-30 Nukleotide langen Fragment von SEQ ID NO:1, 21 oder 73-74;
(ii) einer Nukleinsäure, die mindestens 90% identisch mit einer Nuklein.säure nach (i) ist, oder
(iii) einer Nukleinsäure, die komplementär zu einer Nukleinsäure nach (i) oder (ii) ist,
wobei das Nukleinsäuremolekül nur mit *L. brevis* spezifisch hybridisiert und nicht mit Lactobacillus lindneri, Lactobacillus casel, Lactobacillus paracasei, Lactobacillus coryniformis coryniformis, Lactobacillus corynifortnis torquens, Lactobacillus curvatus, Padiacoccus lamnosus. Pedlococcus inopinatus, Pecünatus cerevisriphilus. Pectinatus frisinglensis, Pectinatus sp. DSM 20462, Megasphaera cerevitiae, Selenomonas lacticifex, Zymophitus paucivorans, Zymophllus raffinosivorans.

10. Nukleinsäuremolekül nach Anspruch 9, **dadurch gekennzeichnet, dass** es eine DNA oder eine RNA ist.

11. Nukleinsäuremolekül nach Anspruch 9, **dadurch gekennzeichnet, dass** es eine PNA ist.

12. Nukleinsäuremolekül nach einem der Ansprüche 9 bis 11, **dadurch gekennzeichnet ist, dass** bis zu 20% der Nukleotide in 10 aufeinanderfolgenden Nukleotiden, insbesondere 1 oder 2 Nukleotide aus dem 10er-Block durch Nukleotide ersetzt sind, die in Bakterien nicht natürlich vorkommen.

13. Kombination aus mindestens zwei Nukleinsäuremolekülen, ausgewählt aus einer Kombination aus mindestens zwei Nukleinsäuremolekülen nach einem der Ansprüche 9 bis 12.

14. Kit, enthaltend ein Nukleinsäuremolekül nach einem der Ansprüche 9 bis 12 und/oder eine Kombination nach Anspruch 13.

15. Verfahren nach einem der Ansprüche 1 bis 8, **dadurch gekennzeichnet, dass** im Schritt (a) eine Kombination aus mindestens zwei Nukleinsäuremoleküten ausgewählt aus
(i) einer Nukleinsäure mit einer Sequenz nach SEQ ID NO 1, SEQ ID NO 21 oder SEQ ID NO 73-74 oder ein mindestens 10, vorzugsweise 15-30 Nukleotide langes Fragment derselben;
(ii) einer Nukleinsäure, die mit einer Nukleinsäure nach (i) spezifisch hybridisiert;
(iii) einer Nukleinsäure, die mindestens 70%, vorzugsweise mindestens 90% identisch mit einer Nukleinsäure nach (i) oder (ii) ist, oder
(iv) einer Nukleinsäure, die komplementär zu einer Nukleinsäure nach (i) bis (iii) ist,
eingesetzt wird, wobei das jeweilige Nukleinsäuremolekül optional
a) eine DNA oder RNA ist,
b) eine PNA ist, oder
c) **dadurch gekennzeichnet ist, dass** bis zu 20% der Nukleotide in 10 aufeinanderfolgenden Nukleotiden, insbesondere 1 oder 2 Nukleotide aus dem 10er-Block, durch Nukleotide ersetzt sind, die in Bakterien nicht natürlich vorkommen.

16. Verfahren nach einem der Ansprüche 1 bis 8 und 15, **dadurch gekennzeichnet, dass** als zweites Nukleinsäuremolekül (Sonde) mindestens ein Nukleinsäuremolekül nach einem der Ansprüche 9 bis 12 eingesetzt wird.

17. Verfahren nach Anspruch 16, **dadurch gekennzeichnet, dass** als zweites oder weiteres Nukleinsäuremolekül (Sonde) mindestens ein Nukleinsäuremolekül mit einer Sequenz nach einer der SEQ ID NO 21 oder SEQ ID NO 73-74 eingesetzt wird.

18. Verwendung eines Nukleinsäuremoleküls ausgewählt aus
(a)
(i) einer Nukleinsäure mit einer Sequenz nach SEQ ID NO 21 oder SEQ ID NO 73-74, oder einem Fragment von SEQ ID NO:1, 21, 73 oder 74 mit 15-30 Nukleotiden,
(ii) einer Nukleinsäure, die mindestens 90% identisch zu einer Nukleinsäure nach (i) ist, und
(iii) einer Nukleinsäure, die komplementär zu einer Nukleinsäure nach (i) bis (ii), ist,
wobei das Nukleinsäuremolekül nur mit *L. brevis* spezifisch hybridisiert und nicht mit Lactobacillus lindneri, Lactobacillus casei. Lactobacillus paracasei, Lactobacillus coryniformis coryniformis, Lactobacillus coryniformis torquens, Lactobacillus curvatus, Pediococcus damnosus. Pediococcus inopinatus, Pectinatus cerevisilphilus, Pectinatus frisingiensis, Pectinatus sp. DSM 20462, Megasphaera cerevisiae, Selenomonas lacticifex, Zymophilus paucivorans, Zymophilus raffinosivorans
zum keimspezifischen Nachweis und/oder Identifizieren und/oder Charakterisieren, von Bakterien der Art *Lactobacillus brevis*; oder
(b) einer Wornbination von mindestens 2 Nukleinsäuren von (a) zum keimspezigschen. Nachweis von L. brevis.

19. Verwendung gemäß Anspruch 18, wobei das Nukleinsäuremolekül eine DNA, RNA oder PNA ist oder **dadurch gekennzeichnet ist, dass** bis zu 20% der Nukleotide in 10 aufeinanderfolgenden Nukleotiden, insbesondere 1 oder 2 Nukleotide aus dem 10er-Block, durch Nukleotide ersetzt sind, die in Bakterien nicht natürlich vorkommen.

## Claims

1. Method for the detection of *Lactobacillus brevis* in a sample; comprising the following steps:
a) bringing the sample into contact with a combination of at least two first nucleic acid molecules (primers), which hybridize with a range of a microbial nucleic acid, conserved in all bacteria of the species Lactobacillus brevis, Lactobacillus lindneri, Lactobacillus casei, Lactobacillus paracasei, Lactobacillus coryniformis, Lactobacillus curvatus, Pediococcus damnosus, Pediococcus inopinafus, Pectinatus cerevisiiphilus, Pectinatus frisingiensis, Pectinatus sp. DSM 20764, Megasphaera cerevisiae, Selenomonas lacticifex, Zymophilus paucivorans, Zymophilus raffinosivorans,
wherein said at least two first nucleic acid molecules are derived from the intergenic spacer 23S-5S or the adjacent 23S or 5S rDNA gene region of *L. brevis,* i.e. as set forth in SEQ ID NO: 1.
b) amplifying the microbial nucleic acid or a portion thereof to produce at least one amplification fragment;
c) bringing the amplification fragments of step b) into contact with at least one second nucleic acid molecule (probe), which specifically hybridizes with at least one amplification fragment comprising a sequence of said microbial nucleic acid specific for bacteria of the species *Lactobacillus brevis*,
wherein said at least one second nucleic acid molecule is derived from the intergenic spacer 23S-5S or the adjacent 23S or 5S-rDNA gene region from *L. brevis*, i.e. as set forth in SEQ ID NO: 1.
d) detecting at least one hybrid nucleic acid consisting of an amplification fragment and a second nucleic acid molecule introduced in step c).

2. Method according to claim 1, **characterized in that** the amplification comprises a polymerase chain reaction (PCR).

3. Method according to Claim 1, **characterised in that** the amplification comprises a ligase chain reaction.

4. Method according to Claim 1, **characterised in that** the amplification comprises an isothermal nucleic acid amplification.

5. Method according to one of Claims 1 to 4, **characterised in that** the second nucleic acid molecule is modified or labelled to produce a detectable signal, the modification or labelling being selected from (i) radioactive groups, (ii) coloured groups, (iii) fluorescent groups, (iv) groups for immobilisation on a solid phase and (v) groups which allow an indirect or directed reaction, particularly by means of antibodies, antigens, enzymes and/or substances with affinity for enzymes or enzyme complexes.

6. Method according to one of the preceding Claims, **characterized in that** the first nucleic acid molecule and/or second nucleic acid molecule are at least 10 nucleotides, preferably 15-30 nucleotides long.

7. Method according to one of the preceding Claims, **characterised in that** the first nucleic acid molecules and/or the second nucleic acid molecule is modified **in that** up to 20% of the nucleotides in 10 consecutive nucleotides, in particular 1 or 2 nucleotides from the block of 10, are replaced by nucleotides which do not naturally occur in bacterial.

8. Method according to one of the preceding Claims, **characterised in that** the conserved region occurs in the genome section which contains the bacterial 23S and 5S genes of bacteria of the species *Lactobacillus brevis.*

9. A nucleic acid molecule suitable as probe for the pathogen specific detection of bacteria of the species *Lactobacillus brevis,* selected from:
(i) a nucleic acid with a sequence according to SEQ ID NO: 21 or SEQ ID Nos: 73-74, or a fragment of 15 to 30 nucleotides of one of SEQ ID Nos:1, 21 or 73 to 74;
(ii) a nucleic acid which is at least 90% identical with a nucleic acid according to (i) or
(iii), a nucleic acid, which is complementary to any nucleic acid according to (i) to (ii),
wherein the nucleic acid molecule hybridizes only specifically with *L. brevis* but not with Lactobacillus brevis, Lactobacillus lindneri, Lactobacillus casei, Lactobacillus paracasei, Lactobacillus coryniformis, Lactobacillus curvatus, Pediococcus damnosus, Pediococcus inopinafus, Pectinatus cerevisiiphilus, Pectinatus frisingiensis, Pectinatus sp. DSM 20764, Megasphaera cerevisiae, Selenomonas lacticifex, Zymophilus paucivorans, Zymophilus raffinosivorans.

10. Nucleic acid molecule according to Claim 9, **characterized in that** it is a DNA or an RNA.

11. Nucleic acid molecule according to Claim 9, **characterised in that** it is a PNA.

12. Nucleic acid molecule according to Claims 9 to 11, **characterised in that** up to 20% of the nucleotides in 10 consecutive nucleotides, in particular 1 or 2 nucleotides from the block of 10, are replaced by nucleotides which do not occur naturally in bacteria.

13. Combination of at least two nucleic acid molecules, selected from a combination of at least two nucleic acid molecules according to any one of claims 9 to 12.

14. Kit comprising a nucleic acid molecule according to any of claims 9 to 12 and/or a combination according to claim 13.

15. A method according to any of claims 1 to 8, **characterized in that** in step (a) a combination of at least two nucleic acid molecules is used, which is selected from:
(i) a nucleic acid having a sequence according to SEQ ID NO:1, SEQ ID NO:21 and SEQ ID NOs: 73-74, or a fragment thereof of at least 10, preferably 15 to 30 nucleotides;
(ii) a nucleic acid, which hybridizes specifically with a nucleic acid according to (i);
(iii) a nucleic acid, which is at least 70%, preferably at least 90% identical with a nucleic acid according to (i) or (ii) or
(iv) a nucleic acid complementary to any nucleic acid according to (i) to (iii),
wherein the respective nucleic acid molecule optionally is,
(a) a DNA or RNA;
(b) a PNA or
(c) **characterized in that** up to 20% of the nucleotides in 10 consecutive nucleotides, in particular 1 or 2 nucleotides of a block of 10, are replaced by nucleotides, which do not naturally occur in bacteria.

16. A method according to any of claims 1 to 8 and 15, **characterised in that** as a second nucleic acid molecule (probe) at least one nucleic acid molecule according to any of claims 9 to 12 is used.

17. Method according to claim 16, **characterised in that** as second or further nucleic acid molecule (probe), at least a nucleic acid molecule is used having a sequence according to any of SEQ ID NOs: 21 or 73 to 74.

18. Use of a nucleic acid molecule selected from:
(a)
(i) a nucleic acid having a sequence according to SEQ ID NO:21 or SEQ ID NOs:73 to 74 or a fragment of SEQ ID NOs: 1, 21, 73 or 74 of 15 to 30 nucleotides;
(ii) a nucleotide acid which is at least 90% identical with a nucleic acid according to (i), and
(iii) a nucleic acid, which is complementary to any nucleic acid according to (i) to (ii),
wherein the nucleic acid molecule hybridizes only specifically with *L. brevis* but not with Lactobacillus brevis, Lactobacillus lindneri, Lactobacillus casei, Lactobacillus paracasei, Lactobacillus coryniformis, Lactobacillus curvatus, Pediococcus damnosus, Pediococcus inopinafus, Pectinatus cerevisiiphilus, Pectinatus frisingiensis, Pectinatus sp. DSM 20764, Megasphaera cerevisiae, Selenomonas lacticifex, Zymophilus paucivorans, Zymophilus raffinosivorans;
for the pathogen specific detection and/or identification and/or characterisation of bacteria of *Lactobacillus brevis*; or
(b) a combination of at least two nucleic acids of (a) for the pathogen specific detection of *L. brevis.*

19. Use according to claim 18, wherein the nucleic acid molecule is a DNA, RNA, PNA or is **characterized in that** up to 20% of the nucleotides in 10 consecutive nucleotides, in particular 1 or 2 nucleotides of a block of 10, are replaced by nucleotides which do not naturally occur in bacteria.

## Revendications

1. Procédé de détection de *Lactobacillus brevis* dans un échantillon, qui comporte les étapes suivantes :
(a) mise en contact de l'échantillon avec une combinaison d'au moins deux premières molécules d'acide nucléique (amorces) qui s'hybrident avec un domaine d'un acide nucléique microbien conservée dans toutes les bactéries des espèces Lactobacillus brevis, Lactobacillus lindneri, Lactobacillus casei, Lactobacillus paracasei, Lactobacillus coryniformis, Lactobacillus curvatus, Pediococcus damnosus, Pediococcus inopinafus, Pectinatus cerevisiiphilus, Pectinatus frisingiensis, Pectinatus sp. DSM 20764, Megasphaera cerevisiae, Selenomonas lacticifex, Zymophilus paucivorans, Zymophilus raffinosivorans ;
dans lequel les au moins deux premières molécules d'acide nucléique sont dérivées de l'espaceur intergénique 23S-5S, respectivement des régions de gènes voisines 23S-ou 5S de l'ADNr du L. brevis, c'est-à-dire comme représenté dans la SEQ ID NO : 1 ;
(b) amplification de l'acide nucléique microbien ou d'une partie de celui-ci pour la production d'au moins un fragment d'amplification ;
(c) mise en contact des fragments d'amplification obtenus dans l'étape (b) avec au moins une deuxième molécule d'acide nucléique (sonde), laquelle s'hybride spécifiquement avec au moins un fragment d'amplification qui comprend une séquence de l'acide nucléique microbien spécifique pour les bactéries du genre *Lactobacillus brevis,*
dans lequel au moins un deuxième acide nucléique est dérivé de l'espaceur intergénique 23S-5S, respectivement des régions de gène voisines 23S ou 5S de l'ADNr du L. brevis, c'est-à-dire comme représenté dans la SEQ ID NO : 1 ;
(d) détection d'au moins un acide nucléique hybride, qui consiste en un fragment d'amplification et en une deuxième molécule d'acide nucléique introduite dans l'étape (c).

2. Procédé selon la revendication 1, **caractérisé en ce que** l'amplification comprend une réaction en chaîne de polymérase (PCR).

3. Procédé selon la revendication 1, **caractérisé en ce que** l'amplification comprend une réaction en chaîne de ligase.

4. Procédé selon la revendication 1, **caractérisé en ce que** l'amplification comprend une amplification isotherme d'acide nucléique.

5. Procédé selon l'une des revendications 1 à 4, **caractérisé en ce que** la deuxième molécule d'acide nucléique est modifiée, respectivement marquée pour la production d'un signal détectable, dans lequel la modification, respectivement le marquage est choisi parmi (i) des groupes radioactifs (ii) des groupes colorés, (iii) des groupes fluorescents, (iv) des groupes pour l'immobilisation sur une phase solide et (v) des groupes qui permettent une réaction indirecte ou directe, en particulier à l'aide d'anticorps, d'antigènes, d'enzymes et/ou de substances avec une affinité pour des enzymes ou des complexes d'enzymes.

6. Procédé selon l'une des revendications précédentes, **caractérisé en ce que** les premières molécules d'acide nucléique et/ou la deuxième molécule d'acide nucléique ont une longueur d'au moins 10 nucléotides, de préférence de 15 à 30 nucléotides.

7. Procédé selon l'une des revendications précédentes, **caractérisé en ce que** les premières molécules d'acide nucléique et/ou la deuxième molécule d'acide nucléique sont modifiées **en ce que** jusqu'à 20 % des nucléotides dans 10 nucléotides successifs, en particulier 1 ou 2 nucléotides en provenance du bloc de 10, sont remplacés par des nucléotides qui ne se retrouvent pas naturellement dans les bactéries.

8. Procédé selon l'une des revendications précédentes, **caractérisé en ce que** le domaine conservé se retrouve dans la section de génome qui contient les gènes 23S- et 5S provenant de bactéries du genre *Lactobacillus brevis.*

9. Molécule d'acide nucléique convenant comme sonde pour la détection spécifique de germe des bactéries du genre *Lactobacillus brevis,* choisie parmi :
(i) un acide nucléique avec une séquence d'après SEQ ID NO : 21 ou SEQ ID NO : 73-74 ou avec un fragment d'une longueur de 15 à 30 nucléotides de SEQ ID NO : 1, 21 ou 73-74 ;
(ii) un acide nucléique qui est identique à au moins 90 % à un acide nucléique d'après (i) ou
(iii) un acide nucléique qui est complémentaire d'un acide nucléique d'après (i) ou (ii),
dans laquelle la molécule d'acide nucléique ne s'hybride spécifiquement qu'avec *L. brevis* et non avec Lactobacillus lindneri, Lactobacillus casei, Lactobacillus paracasei, Lactobacillus coryniformis coryniformis, Lactobacillus coryniformis torquens, Lactobacillus curvatus, Pediococcus damnosus, Pediococcus inopinatus, Pectinatus cerevisiiphilus, Pectinatus frisingiensis, Pectinatus sp. DSM 20462, Megasphaera cerevisiae, Selenomonas lacticifex, Zymophilus paucivorans, Zymophilus raffinosivorans.

10. Molécule d'acide nucléique selon la revendication 9, **caractérisée en ce que** c'est un ADN ou un ARN.

11. Molécule d'acide nucléique selon la revendication 9, **caractérisée en ce que** c'est un PNA.

12. Molécule d'acide nucléique selon l'une des revendications 9 à 11, **caractérisée en ce que** jusqu'à 20 % des nucléotides dans 10 nucléotides consécutifs, en particulier 1 ou 2 nucléotides du bloc de 10, sont remplacés par des nucléotides qui ne se retrouvent pas naturellement dans les bactéries.

13. Combinaison d'au moins deux molécules d'acide nucléique, choisies parmi une combinaison d'au moins deux molécules d'acide nucléique selon une des revendications 9 à 12.

14. Kit contenant une molécule d'acide nucléique selon une des revendications 9 à 12 et/ou une combinaison selon la revendication 13.

15. Procédé selon une des revendications 1 à 8, **caractérisé en ce que** l'on utilise dans l'étape (a) une combinaison d'au moins deux molécules d'acide nucléique, choisies parmi
(i) un acide nucléique avec une séquence selon SEQ ID NO : 1, SEQ ID NO : 21 ou SEQ ID NO : 73-74 ou un fragment d'une longueur d'au moins 10, de préférence de 15 à 30 nucléotides de celui-ci ;
(ii) un acide nucléique qui s'hybride spécifiquement avec une acide nucléique selon (i)
(iii) un acide nucléique qui est identique à au moins 70 %, de préférence à au moins 90 % à un acide nucléique selon (i) ou (ii), ou
(iv) un acide nucléique qui est complémentaire à un acide nucléique selon (i) à (iii),
dans lequel la molécule respective d'acide nucléique est en option
a) un ADN ou un ARN
b) un PNA, ou
c) est **caractérisée en ce que** jusqu'à 20 % des nucléotides dans 10 nucléotides successifs, en particulier 1 ou 2 nucléotides du bloc de 10, sont remplacés par des nucléotides qui ne se retrouvent pas naturellement dans des bactéries.

16. Procédé selon l'une des revendications 1 à 8 et 15, **caractérisé en ce que**, comme deuxième nucléotide (sonde), on utilise au moins une molécule d'acide nucléique selon l'une des revendications 9 à 12.

17. Procédé selon la revendication 16, **caractérisé en ce que**, comme deuxième nucléotide ou comme nucléotide suivant (sonde), on utilise au moins une molécule d'acide nucléique avec une séquence selon une des SEQ ID NO : 21 ou SEQ ID NO : 73-74.

18. Utilisation d'une molécule d'acide nucléique, choisie parmi
(a)
(i) un acide nucléique avec une séquence d'après SEQ ID NO : 21 ou SEQ ID NO : 73-74 ou avec un fragment d'une longueur de 15 à 30 nucléotides de SEQ ID NO : 1, 21, 73 ou 74 ;
(ii) un acide nucléique qui est identique à au moins 90 % à un acide nucléique d'après (i), et
(iii) un acide nucléique qui est complémentaire d'un acide nucléique d'après (i) ou (ii),
dans laquelle la molécule d'acide nucléique ne s'hybride spécifiquement qu'avec *L. brevis* et non avec Lactobacillus lindneri, Lactobacillus casei, Lactobacillus paracasei, Lactobacillus coryniformis coryniformis, Lactobacillus coryniformis torquens, Lactobacillus curvatus, Pediococcus damnosus, Pediococcus inopinatus, Pectinatus cerevisiiphilus, Pectinatus frisingiensis, Pectinatus sp. DSM 20462, Megasphaera cerevisiae, Selenomonas lacticifex, Zymophilus paucivorans, Zymophilus raffinosivorans
pour la détection spécifique de germe et/ou l'identification et/ou la caractérisation de bactéries du genre *Lactobacillus brevis* ; ou
(b) une combinaison d'au moins 2 acides nucléiques de (a) pour la détection spécifique de germe de L. brevis.

19. Utilisation selon la revendication 18, dans laquelle la molécule d'acide nucléique est un ADN, un ARN ou un PNA ou **caractérisée en ce que** jusqu'à 20 % des nucléotides dans 10 nucléotides successifs, en particulier 1 ou 2 nucléotides du bloc de 10, sont remplacés par des nucléotides qui ne se retrouvent pas naturellement dans des bactéries.
